# EUROPEAN PATENT SPECIFICATION

(11) **EP 1 192 182 B1**
(45) Date of publication and mention of the grant of the patent: **04.06.2008**
(21) Application number: 00967002.7
(22) Date of filing: 27.09.2000
(51) Int. Cl.: C07K 14/575, C12N 15/16, C12N 15/10, C12N 15/67, A61K 48/00, A61K 38/22, A61P 3/08

(54) **PITUITARY ADENYLATE CYCLASE ACTIVATING PEPTIDE (PACAP) RECEPTOR 3 (R3) AGONISTS AND THEIR PHARMACOLOGICAL METHODS OF USE**
AGONISTEN DES REZEPTORS 3 (R3) VON PITUITARY ADENYLATE CYCLASE AKTIVIERENDEM PEPTID (PACAP) UND IHRE PHARMAKOLOGISCHE VERWENDUNG
RECEPTEURS 3 AGONISTES (R3) PITUITAIRE ADENYL CYCLASE ACTIVATEUR DE PEPTIDE (PACAP) ET LEURS PROCEDES PHARMACOLOGIQUES D'UTILISATION

(30) Priority: 28.09.1999 US 407832; 15.06.2000 US 595280
(43) Date of publication of application: 03.04.2002
(73) Proprietor: BAYER CORPORATION, Berkeley, CA 94701-1986 (US)
(72) Inventor: PAN, Clark, Castro Valley, CA 94552 (US); TSUTSUMI, Manami, Stratford, CT 06615 (US); SHANAFELT, Armen, B., Moraga, CA 94556 (US)
(74) Representative: Wain, Christopher Paul
(86) International application number: PCT/US2000/026638
(87) International publication number: WO 2001/023420

(56) References cited:
- EP-A- 0 796 867
- WO-A-00/05260
- WO-A-91/14786
- WO-A-98/02453
- WO-A-99/47159
- US-A- 5 677 419
- GOURLET PHILIPPE ET AL: "Vasoactive intestinal peptide modification at position 22 allows discrimination between receptor subtypes." EUROPEAN JOURNAL OF PHARMACOLOGY, vol. 348, no. 1, 1 May 1998 (1998-05-01), pages 95-99, XP000989782 ISSN: 0014-2999 cited in the application
- INAGAKI NOBUYA ET AL: "Cloning and functional characterization of a third pituitary adenylate cyclase-activating polypeptide receptor subtype expressed in insulin-secreting cells." PROCEEDINGS OF THE NATIONAL ACADEMY OF SCIENCES OF THE UNITED STATES, vol. 91, no. 7, 1994, pages 2679-2683, XP002041195 1994 ISSN: 0027-8424 cited in the application
- GOURLET P ET AL: "THE LONG-ACTING VASOACTIVE INTESTINAL POLYPEPTIDE AGONIST RO25-1553IS HIGHLY SELECTIVE OF THE VIP2 RECEPTOR SUBCLASS" PEPTIDES,US,ELMSFORD, vol. 18, no. 3, 1997, pages 403-408, XP002049505 ISSN: 0196-9781 cited in the application
- ROBBERECHT P ET AL: "INTERET DES RECEPTEURS RECOMBINANTS DANS L'ELABORATION D'AGONISTES ET D'ANTAGONISTES DES RECEPTEURS DU VIP ET DU PACAP. INTEREST OF RECOMBINANT RECEPTORS FOR THE RESTING OF VIP/PACAP RECEPTORS AGONISTS AND ANTAGONISTS" JOURNAL DE PHARMACIE DE BELGIQUE, 1 May 1996 (1996-05-01), XP002049504 cited in the application
- NOKIHARA ET AL: "RECEPTOR RECOGNITION OF PACAP AND VIP EXAMINED BY BINDING STUDIES CAMP PRODUCTION AND BIOLOGICAL ACTIONS BOTH IN VIVO AND IN VITRO BY MEANS OF SELECTIVE RESIDUE SUBSTITUTION" EDINBURGH, SEPT. 8 - 13, 1996,WEST MIDLANDS: MAYFLOWER SCIENTIFIC,GB, vol. SYMP. 24, 1996, pages 63-66, XP002153330 ISBN: 0-9527011-2-X
- GOURLET P ET AL: "ADDITION OF THE (28-38) PEPTIDE SEQUENCE OF PACAP TO THE VIP SEQUENCE MODIFIES PEPTIDE SELECTIVITY AND EFFICACY" INTERNATIONAL JOURNAL OF PEPTIDE AND PROTEIN RESEARCH,DK,MUNKSGAARD, COPENHAGEN, vol. 48, no. 4, 1 October 1996 (1996-10-01), pages 391-396, XP000628828 ISSN: 0367-8377 cited in the application
- GOURLET P ET AL: "Analogues of VIP, helodermin and PACAP discriminate between rat and human VIP1 and VIP2 receptors." ANNALS OF THE NEW YORK ACADEMY OF SCIENCES, vol. 865, 11 December 1998 (1998-12-11), pages 247-252, XP000989781 3rd International Symposium;Freiburg, Germany; September 17-20, 1997, peptides. Dec. 11, 1998 New York Academy of Sciences 2 East 63rd Street, New York, New York 10021, USA ISBN: 1-57331-153-7 cited in the application
- GOURLET P ET AL: "C-TERMINALLY SHORTENED PITUITARY ADENYLATE CYCLASE-ACTIVATING PEPTIDES (PACAP) DISCRIMINATE PACAP I, PACAP II-VIP1 AND PACAP II-VIP2 RECOMBINANT RECEPTORS" REGULATORY PEPTIDES,NL,ELSEVIER SCIENCE BV, vol. 62, no. 2/03, 1996, pages 125-130, XP000909505 ISSN: 0167-0115
- DICKINSON T ET AL: "VIP and PACAP: very important in pain?" TRENDS IN PHARMACOLOGICAL SCIENCES,GB,ELSEVIER TRENDS JOURNAL, CAMBRIDGE, vol. 20, no. 8, 1 August 1999 (1999-08-01), pages 324-329, XP004173657 ISSN: 0165-6147
- HARMAR ANTHONY J ET AL: "International union of pharmacology. XVIII. Nomenclature of receptors for vasoactive intestinal peptide and pituitary adenylate cyclase-activating polypeptide." PHARMACOLOGICAL REVIEWS, vol. 50, no. 2, June 1998 (1998-06), pages 265-270, XP000989676 ISSN: 0031-6997 cited in the application
- POHL MARKUS ET AL: "Molecular cloning of the helodermin and exendin-4 cDNAs in the lizard: Relationship to vasoactive intestinal polypeptide/pituitary adenylate cyclase activating polypeptide and glucagon-like peptide 1 and evidence against the existence of mammalian homologues." JOURNAL OF BIOLOGICAL CHEMISTRY, vol. 273, no. 16, 17 April 1998 (1998-04-17), pages 9778-9784, XP002162243 ISSN: 0021-9258

## Description

### Field of the Invention

This invention relates to newly identified polypeptides and the use of such polypeptides for therapeutic purposes. More particularly, the polypeptides of the present invention are useful in stimulating the release of insulin from pancreatic beta cells in a glucose-dependent manner, thereby providing a treatment option for those individuals afflicted with a metabolic disorder such as diabetes or impaired glucose tolerance, a prediabetic state.

### Background of the Related Art

Diabetes is characterized by impaired glucose metabolism manifesting itself among other things by an elevated blood glucose level in the diabetic patient. Underlying defects lead to a classification of diabetes into two major groups: type I diabetes, or insulin dependent diabetes mellitus (IDDM), which arises when patients lack beta-cells producing insulin in their pancreatic glands, and type 2 diabetes, or non-insulin dependent diabetes mellitus (NIDDM), which occurs in patients with an impaired beta-cell function and alterations in insulin action.

Type I diabetic patients are currently treated with insulin, while the majority of type 2 diabetic patients are treated with agents that stimulate beta-cell function or with agents that enhance the tissue sensitivity of the patients towards insulin. Over time almost one-half of type 2 diabetic subjects lose their response to these agents and then must be placed on insulin therapy. The drugs presently used to treat type 2 Diabetes include:
Alpha-glucosidase inhibitors (PRECOSE®, VOGLIBOSE^{™}, and MIGLITOL®). Alpha-glucosidase inhibitors reduce the excursion of postprandial glucose by delaying the absorption of glucose from the gut. These drugs are safe and provide treatment for mild to moderately affected diabetic subjects. However, gastrointestinal side effects have been reported in the literature.
Insulin sensitizers. Insulin sensitizers are drugs that enhance the body's response to insulin. Thiozolidinediones such as REZULIN^{™} (troglitazone) activate the PPAR gamma receptor and modulate the activity of a set of genes that have not been well described. Although effective, these drugs have been associated with liver toxicity. Because of hepatotoxicity, REZULIN has been withdrawn from the market.
Insulin secretagogues (sulfonylureas and other agents that act by the ATP-dependent K+ channel). SFUs are standard therapy for type 2 diabetics that have mild to moderate fasting glycemia. The SFUs have limitations that include a potential for inducing hypoglycemia, weight gain, and high primary and secondary failure rates. 10 to 20% of initially treated patients fail to show a significant treatment effect (primary failure). Secondary failure is demonstrated by an additional 20-30% loss of treatment effect after six months on an SFU. Insulin treatment is required in 50% of the SFU responders after 5-7 years of therapy (Scheen, A.J., et al., Diabetes Res. Clin. Pract. 6:533-543 (1989)).

GLUCOPHAGE^{™} (metformin HCl) is a biguanide that lowers blood glucose by decreasing hepatic glucose output and increasing peripheral glucose uptake and utilization. The drug is effective at lowering blood glucose in mildly and moderately affected subjects and does not have the side effects of weight gain or the potential to induce hypoglycemia. However, GLUCOPHAGE has a number of side effects including gastrointestinal disturbances and lactic acidosis. GLUCOPHAGE is contraindicated in diabetics over the age of 70 and in subjects with impairment in renal or liver function. Finally, GLUCOPHAGE has the same primary and secondary failure rates as the SFUs.

Insulin treatment is instituted after diet, exercise, and oral medications have failed to adequately control blood glucose. This treatment has the drawbacks that it is an injectable, that it can produce hypoglycemia, and that it causes weight gain.

Because of the problems with current treatments, new therapies to treat type 2 diabetes are needed. In particular, new treatments to retain normal (glucose-dependent) insulin secretion are needed. Such new drugs should have the following characteristics: dependent on glucose for promoting insulin secretion, i.e. produce insulin secretion only in the presence of elevated blood glucose; low primary and secondary failure rates; and preserve islet cell function. The strategy to develop the new therapy disclosed herein is based on the cyclic adenosine monophosphate (cAMP) signaling mechanism and its effects on insulin secretion.

Cyclic AMP is a major regulator of the insulin secretion process. Elevation of this signaling molecule promotes the closure of the K+ channels following the activation of protein kinase A pathway. Closure of the K+ channels causes cell depolarization and subsequent opening of Ca++ channels, which in turn leads to exocytosis of insulin granules. Little if any effects on insulin secretion occurs in the absence of low glucose concentrations (Weinhaus, A., et al., Diabetes 47: 1426-1435 (1998)). Secretagogues like pituitary adenylate cyclase activating peptide ("PACAP") and GLP-1 use the cAMP system to regulate insulin secretion in a glucose-dependent fashion (Komatsu, M., et al., Diabetes 46: 1928-1938, (1997)). Insulin secretagogues working through the elevation of cAMP such as GLP-1 and PACAP is also able to enhance insulin synthesis in addition to insulin release (Skoglund, G. et al., Diabetes 49: 1156-1164, (2000). Borboni, P. et al., Endocrinology 140: 5530-5537, (1999)).

PACAP is a potent stimulator of glucose-dependent insulin secretion from pancreatic beta-cells. Three different PACAP receptor types (R1, R2, and R3) have been described (Harmar, A. et al., Pharmacol. Reviews 50: 265-270 (1998)). PACAP displays no receptor selectivities, having comparable activities and potencies at all three receptors. R1 is located predominately in the CNS, whereas R2 and R3 are more widely distributed. R2 is located in the CNS as well as in liver, lungs and intestine. R3 is located in the CNS, pancreas, skeletal muscle, heart, kidney, adipose tissue, testis and stomach. Recent work argues that R3 is responsible for the insulin secretion from beta cells (Inagaki, N. et al., PNAS 91: 2679-2683, (1994)). This insulinotropic action of PACAP is mediated by the GTP binding protein Gs. Accumulation of intracellular cAMP in turn activates the nonselective cation channels in beta cells increasing [Ca++], and promotes exocytosis of insulin-containing secretory granules.

PACAP is the newest member of the superfamily of metabolic, neuroendocrine and neurotransmitter peptide hormones that exert their action through the cAMP-mediated signal transduction pathway (Arimura, Regul. Peptides 37:287-303 (1992)). The biologically active peptides are released from the biosynthetic precursor in two molecular forms, either as a 38-amino acid peptide (PACAP-38) and/or as a 27-amino acid peptide (PACAP-27) with an amidated carboxyl termini (Arimura, supra).

The highest concentrations of the two forms of the peptide are found in the brain and testis (reviewed in Arimura, supra). The shorter form of the peptide, PACAP-27, shows 68% structural homology to vasoactive intestinal polypeptide (VIP). However, the distribution of. PACAP and VIP in the central nervous system suggests that these structurally related peptides have distinct neurotransmitter functions (Koves et al., Neuroendocrinology 54:159-169, (1991)).

Recent studies have demonstrated diverse biological effects of PACAP-38, from a role in reproduction (McArdle, Endocrinology 135:815-817 (1994)) to ability to stimulate insulin secretion (Yada et al., J. Biol. Chem. 269:1280-1293 (1994)).

Vasoactive intestinal peptide (VIP) is a 28 amino acid peptide that was first isolated from hog upper small intestine (Said and Mutt, Science 169: 1217-1278, 1970; U.S. Patent No. 3,879.371). This peptide belongs to a family of structurally-related, small polypeptides that includes helodermin, secretin, the somatostatins, and glucagon. The biological effects of VIP are mediated by the activation of membrane-bound receptor proteins that are coupled to the intracellular cAMP signaling system. These receptors were originally known as VIP-R1 and VIP-R2, however, they were later found to be the same receptors as PACAP-R2 and PACAP-R3. VIP displays comparable activities and potencies at PACAP-R2 and PACAP-R3.

To improve the stability of VIP in human lung fluid, Bolin et al (Biopolymers 37: 57-66, (1995)) made a series of VIP variants designed to enhance the helical propensity of this peptide and reduce proteolytic degradation. Substitutions were focused on positions 8. 12, 17, and 25-28, which were implicated to be unimportant for receptor binding. Moreover, the "GGT" sequence was tagged onto the C-terminus of VIP muteins with the hope of more effectively capping the helix. Finally, to further stabilize the helix, several cyclic variants were synthesized (US. Patent No. 5,677,419). Although these efforts were not directed toward receptor selectivity, they yielded two analogs (designated herein as R3P0 and R3P4) that have greater than 100-fold PACAP-R3 selectivity (Gourlet et al., Peptides 18: 403-408, (1997); Xia et al., J. Pharmacol. Exp. Ther., 281: 629-633, (1997)).

GLP-1 is released from the intestinal L-cell after a meal and functions as an incretin hormone (i.e. it potentiates glucose-induced, insulin release from the pancreatic beta-cell). It is a 37-amino acid peptide, that is differentially expressed by the Glucagon gene, depending upon tissue type. The clinical data that support the beneficial effect of raising cAMP levels in β-cells have been collected with GLP-1. Infusions of GLP-1 in poorly controlled type 2 diabetics normalized their fasting blood glucose levels (Gutniak, M., et al., New Eng. J. Med. 326:1316-1322. (1992)) and with longer infusions improved the beta cell function to those of normal subjects (Rachman, J. et al., Diabetes 45: 1524-1530, (1996)). A recent report has shown that GLP-1 improves the β-cells' ability to respond to glucose in subjects with impaired glucose tolerance (Byme M., et al., Diabetes 47: 1259-1265 (1998)). All of these effects, however, are short-lived because of the short half-life of the peptide. Recently Novo Nordisk has discontinued clinical trials with GLP-1. This failure reportedly was due to a very short plasma half-life of the peptide of a few minutes.

EXENDIN 4^{™}. Amylin Pharmaceuticals is conducting Phase I trials with EXENDIN 4 (AC2993), a 39 amino acid peptide originally identified in Gila Monster. Phase II trials have recently begun. Amylin claims preclinical results showing a 4 hour duration of efficacy and efficacy in animal models when AC2993 is administered subcutaneously, orally, and nasally. However, at doses of 0.2 and 0.3µg/kg, the incidence of headaches, postural hypotension, nausea and vomiting was significant.

US 5,677,419 relates to cyclised analogues of VIP. However, there is no discussion of the selectivity of these analogues for any of the VIP receptors.

Gourlet et al (European Journal of Pharmacology 348 (1998); pages 95 to 99) disclose VIP variants wherein amino acids at positions 22 and 24 have been mutated. They reported that mutations at these positions produced VIP variants that had either no receptor selectivity as compared to VIP, or which had lower affinity for the VIP2 (R3) receptor than VIP.

WO 98/02453 discloses peptides having a higher selectivity for the VIP1 (R2) receptor than for the VIP2 (R3) receptor.

Gourlet et al (Peptides, volume 18, No. 3, pages 403-408 (1997)) disclose and characterise a single VIP receptor polypeptides agonist, RO25-1553.

There exists a need for an improved peptide that has the glucose-dependent insulin secretagogue activity of PACAP, GLP-1, or EXENDIN 4, and yet has fewer side-effects.

This invention provides novel polypeptides that function *in vivo* as agonists of the PACAP R3 receptor (hereafter, R3) and are effective in the treatment of diseases and conditions that can be ameliorated by agents having R3 agonist activity. Preferably, the polypeptides of this invention are selective R3 agonists, having greater potency at R3 than at R2 and R1. For example, but not by way of limitation, these polypeptides stimulate insulin synthesis and release from pancreatic beta cells in a glucose-dependent fashion and subsequent plasma glucose reduction. These insulin secretagogue polypeptides are shown to stimulate Insulin release in rat and human islet cells in *vitro* and *in vivo.* Unlike PACAP-27, these secretagogue polypeptides also lower blood gluclose *in vivo* more than vehicle control upon glucose challenge.

The polypeptides of the present invention provide a new therapy for patients with, for example, metabolic disorders such as those resulting from decreased endogenous insulin secretion, in particular type 2 diabetics, or for patients with impaired glucose tolerance, a prediabetic state that has a mild alteration in insulin secretion.

In particular, one aspect of the invention is a polypeptide selected from the group consisting of SEQ ID NOs: 47, 72 to 82 and 85 and fragments, derivatives and variants thereof that demonstrate at least one biological function that is substantially the same as the polypeptides of the listed SEQ ID Nos. (collectively, "polypeptides of this invention") including functional equivalents thereof. A preferred embodiment of this invention is a polypeptide SEQ ID NO: 72 and fragments, derivatives, and variants thereof that demonstrate at least one biological function that is substantially the same as the polypeptides of the listed SEQ ID NOs.

Another embodiment of the invention is a polynucleotide that encodes for the polypeptides of this invention, and the attendant vectors and host cells necessary to recombinantly express the polypeptides of this invention. These polynucleotide sequences and sequences which are not part of the invention include those identified as SEQ ID Nos. 204, 207 to 211, 214 to 230, and 232 to 321.

The invention also provides pharmaceutical compositions comprising a polypeptide according to the invention with a pharmaceutically acceptable carrier.

The invention also provides gene therapy compositions comprising a polynucleotide according to the invention in combination with a therapeutically-effective gene therapy carrier.

Antibodies and antibody fragments that selectively bind the polypeptides of this invention are also provided. Such antibodies are useful in detecting the polypeptides of this invention, and can be identified and made by procedures well known in the art, including those analogous to that described in Example 17 below.

The invention is also directed to a method of treating diabetes and/or other diseases or conditions affected by the polypeptides of this invention, preferably effected by the R3 agonist function of the polypeptides of this invention, In a mammal, comprising administering a therapeutically effective amount of any of the polypeptides of the present invention to said mammal.

Also disclosed are methods of making the polypeptides of this invention, both recombinant and synthetic.

### BRIEF DESCRIPTION OF THE DRAWING

FIG. 1 depicts amino acid sequences of polypeptides of SEQ ID NO: 11 through 14, SEQ ID NO: 18, SEQ ID NOs: 21 through 26, SEQ ID NOs: 32 through 36. SEQ ID NOs: 40 through 53, SEQ ID NOs: 57 through 61, SEQ ID NOs: 63 through 99, SEQ ID NOs: 102 through 119, SEQ ID NOs: 121 through 137, SEQ ID NOs: 139 through 177, SEQ ID NOs: 179, 180, SEQ ID NOs: 183 through 202, and SEQ ID NOs: 322 through 341 which are claimed polypeptides.

FIG. 2 is a sequence alignment of VIP mutants and native polypeptides VIP, PACAP38. GLP-1. EXENDIN-4 and examples of R3 selective polypeptides. Conserved residues are bolded and shaded in dark gray while conservative changes are shaded in light gray,

FIG. 3 is a restriction map of a typical plasmid containing the GST-peptide fusion.

FIGS. 4A-4B are graphs showing the effects of GLP-1 or R3P3 on insulin release from rat islets *in vitro*.

FIG. 5 is a graph showing the R3P3 peptide effect on glucose disposal.

FIG. 6 is a bar chart showing the effect of PACAP and related polypeptides on intestinal water content of Balb/C mice.

FIG. 7 is a bar chart showing that 1 nmole/kg dose of R3P3, R3P12, R3P13, or GLP-1 enhance glucose disposal in the rat by subcutaneous route of administration.

FIG 8 depicts polynucleotide sequences SEQ ID NOs 54 through 56 and 203 through 301 that encode for the polypeptides of this invention.

The first 6 nucleotides represent the Bam HI restriction enzyme recognition site followed by the next 12 nucleotides which encode the "IEGR" Factor Xa recognition site. The last 6 nucleotides represent the Xho I or Eco RI restriction enzyme recognition site preceded by the 6 nucleotides that encode the two stop codons. The nucleotides between the Factor Xa site and stop codons encode the amino acid sequence of the corresponding polypeptide. The nucleotides between the two restriction sites are cloned Into the corresponding restriction sites on the pGEX-6P-1 vector (Amershm Pharmacia Biotech). The SEQ ID NOs are denoted in parenthesis.

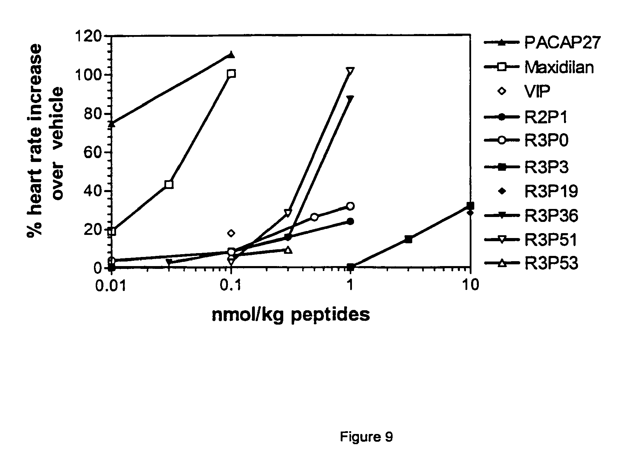

FIG 9 shows the effect of PACAP-27. VIP and receptor selective agonists on the heart rate in conscious dogs (see example 15).

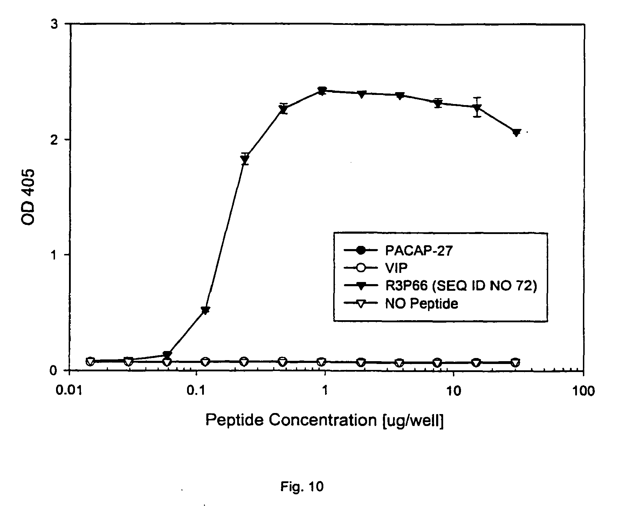

FIG. 10 shows detection of R3P66 by polyclonal antibodies produced in rabbits immunized with R3P66 C-terminus sequence (Ac-CRKOVAAKKYLQSIKNKRY-COOH), using ELISA.

### DETAILED DESCRIPTION OF THE PREFERRED EMBODIMENTS

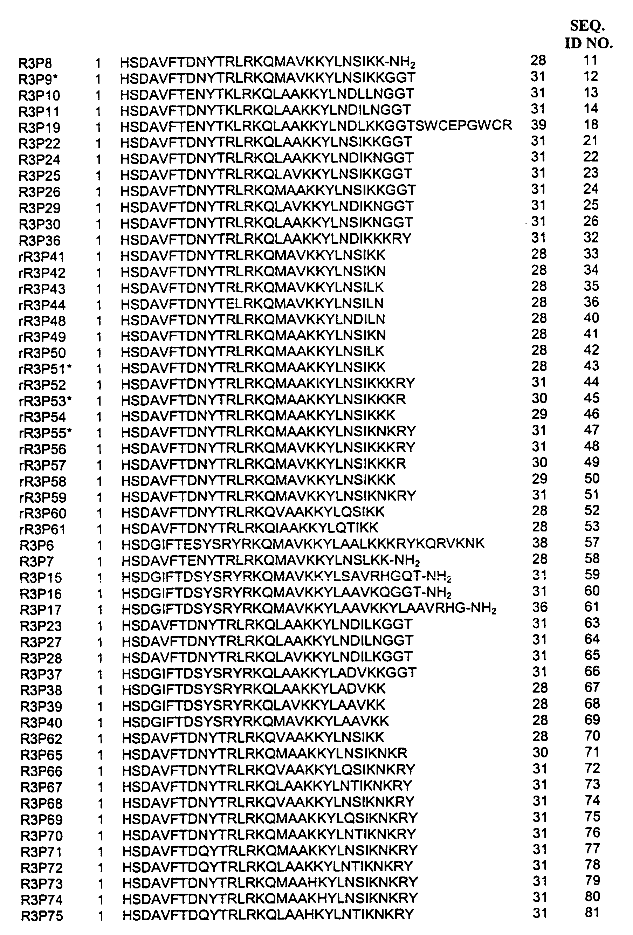

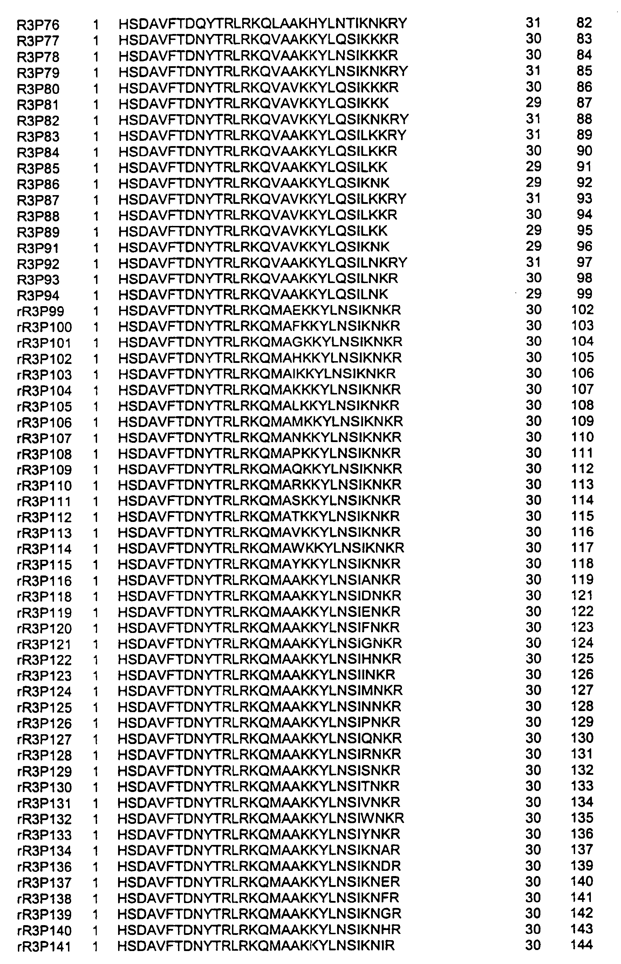

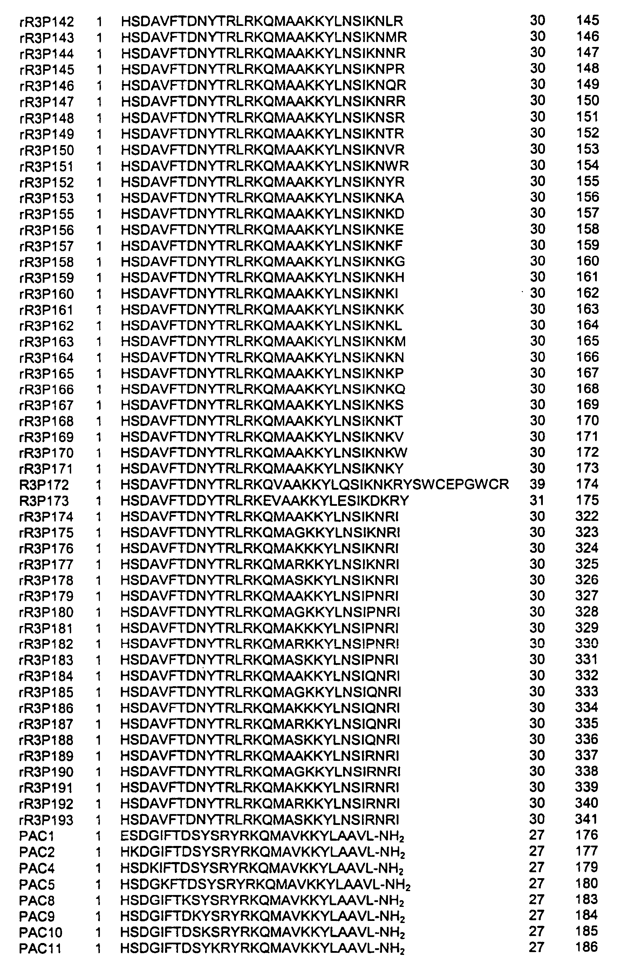

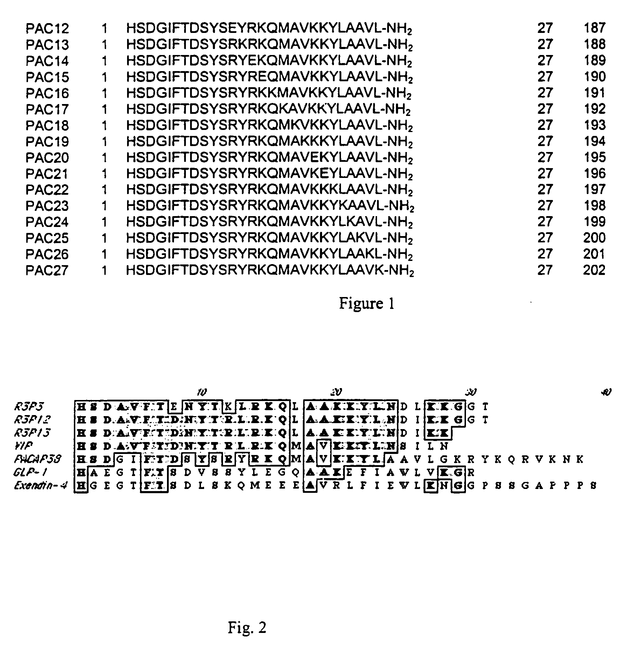

This invention provides novel polypeptides, and fragments, derivative and variants thereof that demonstrate at least one biological function that is substantially the same as the polypeptides of Fig. 1 (collectively, polypeptides of this invention). The polypeptides of this invention function in *vivo* as R3 agonists or otherwise In the prevention and/or treatment of such diseases or conditions as diabetes, asthma, hypertension, male reproduction problems including human sperm motility, cardiovascular diseases, ulcers, and other conditions identified herein, or function otherwise as described later herein. Preferably, the polypeptides of this invention will stimulate insulin release from pancreatic beta cells in a glucose-dependent fashion,

The polypeptides of this invention are R3 agonists. Preferably, they are selective R3 agonists with at least 10-fold selectivity for R3 over R2 and/or R1. More preferably, they are selective R3 agonists with at least 100-fold selectivity for R3 over R2 and/or R1. Most preferably, they stimulate insulin release into plasma in a glucose-dependent fashion without inducing a stasis or increase in the level of plasma glucose that is counterproductive to the treatment of, for example, type 2 diabetes. Additionally, it is preferable for the polypeptides of this invention to be selective agonists of the R3 receptor, thereby causing, for example, an increase In insulin release into plasma, while being selective against other receptors that are responsible for such disagreeable or dangerous side effects as gastrointestinal water retention, and/or unwanted cardiovascular effects such as increased heart rate. It has now been discovered that R3-mediated insulin secretion does not cause hypoglycemia, R2 activation leads to glucose release Into plasma which is counterproductive to the treatment of type 2 diabetes and gastrointestinal water retention, and R1 activation leads to cardiovascular effects such as increased heart rate.

The polypeptides of this invention provide a new therapy for patients with decreased endogenous insulin secretion or impaired glucose tolerance in particular type 2 diabetes.

### A. Discussion

PACAP, VIP, GLP-1 and Exendin-4 are polypeptides capable of stimulating Insulin release In a glucose-dependent fashion. However, this fact alone does not guarantee glucose reduction *in vivo.* Since PACAP is known to bind to PACAP-R1, -R2 and -R3 receptors, and VIP is known to bind to PACAP-R2 and -R3 receptors, it was thought that they may have similar conserved structural features. The stacking alignment below shows the primary structural relationships:

| | | SEQ ID NO. | |
|---|---|---|---|
| VIP | 1 HSDAVFTDNY TRLRKQMAVK KYLNSILN-NH₂ | 28 | 1 |
| PACAP38 | 1 HSDGIFTDSY SRYRKQMAK KYLAAVLGKR YKQRVKNK-NH₂ | 38 | 2 |
| GLP-1 | 1 HAEGTFTSDV SSYLEGQAAK EFIAWLKGR-NH₂ | 30 | 3 |
| Exendin 4 | 1 HGEGTFTSDL SKQMEEEVR LFIEWLKNGG PSSGAPPPS-NH₂ | 39 | 4 |

(where single-letter abbreviations for amino acids can be found in Zubay, Biochemistry 2d ed., 1988. MacMillan Publishing, New York, p. 33), and are defined below. The polypeptides of the present invention and polypeptides which are not part of the invention (Fig. 1) are most closely related to VIP in terms of their primary structure with the exception of SEQ ID NO: 57-61, 66-69, and 176, 177, 179, 180, 183-202 which are more closely related to PACAP.

The inventors herein have created a new polypeptide that is an R3 agonist, preferably a selective R3 agonist, and/or that exhibits a selective glucose-dependent insulin secretagogue effect wherein selective activation of the PACAP R3 receptor indeed leads to a glucose dependent pathway to insulin secretion by pancreatic beta cells, with concomitant glucose reduction *in vivo.* In that light, they first studied the structures of PACAP-27 and VIP in an effort to determine the residues most likely responsible for receptor selectivity. It is known that PACAP and VIP do not reduce glucose *in vivo,* but, rather, they stimulate glucose release from the liver. It has been shown that activation of R2 increases plasma glucose levels *in vivo.* Previously, both PACAP and VIP have been mutagenized extensively for various reasons. For instance, serial deletions of PACAP27 and PACAP38 from both ends confirmed the importance of both termini for receptor binding (Gourlet et al., Eur. J. Pharm. 287: 7-11, (1995); Gourlet et al., Regul. Peptides 62: 125-130, (1996)). Rat brain membrane binding and adenylate cyclase activities of PACAP27/VIP hybrid muteins implicated the importance of the N-terminal residues of PACAP for PACAP-R1 recognition (Ando et al., Biomed. Pept. Proteins Nucleic Acids 2:41-46, (1996)). Increasing the basicity of Leu¹⁷-PACAP27 and Leu¹⁷-VIP by making K15R, K20R, and K21R mutation and extension of the C-terminus with "GKR" sequence led to an increase in duration of guinea pig tracheal relaxant activity proposed to be due to protection from heparin binding (Kashimoto et al., Ann. NY Acad. Sci. 805: 505-510, (1996)). Gourlet et al. (Biochim. Biophys. Acta 1314: 267-273, (1996)) demonstrated that the Q16R mutein of VIP and PACAP possessed greater affinities than their respective native polypeptides for PACAP-R2 and R1, respectively. Gourlet et al. (Peptides 18: 1539-1545, (1997)) developed a high affinity R2-selective agonist by making the chimeric substituted peptide [K15, R16, L27] VIP(1-7)/GRF(8-27), N-terminal acylation and D-Phe2 substitution of this selective agonist led to a potent R2 selective antagonist (Gourlet et al., Peptides 18 1555-1560. (1997)). VIP muteins Y22L and Y22A, but not Y22F, display lower affinity for PACAP-R3, suggesting the importance of an aromatic group at position 22 for receptor R3 binding but not for receptor R2 binding (Gourlet, Eur, J. Biochem. 348: 95-99, (1998)). Helodermin and helospectin, VIP-like peptides isolated from the salivary gland venom of lizards exhibit -100-fold PACAP-R3 selectivity (Gourlet, Ann. NY Acad. Sci. 865: 247-252, (1998)). Photoaffinity labeling of PACAP27 by replacing F6 and Y22 with p-benzoyl-L-phenylaianine (pBz) or K15, K20, and K21 with pBz₂ suggested that K15 and F22 are closer to PACAP-R1 than F6, K20, and K21 (Cao et al., Eur. J. Biochem. 244: 400-406, (1997); Cao et al., Ann. NY Acad. Sci., 865: 82-91, (1998)).

The inventors herein have found several polypeptides that cause the stimulation of release of insulin in a glucose-dependent manner and cause glucose reduction *in vivo.* Those polypeptides and polypeptides which are not part of the present invention bear some similarity to VIP and PACAP. In particular, a stacked alignment shows the following:

| | | | SEQ ID NO. |
|---|---|---|---|
| VIP | 1 HSDAVFTDNY TRLRKQMAVK KYLNSILN-NH₂ | 28 | 1 |
| PACAP38 | 1 HSDGIFTDSY SRYRKQMAVK KYLAAVLGKRYKQRVKNK-NH₂ | 38 | 2 |
| R3P1 | Ac-HSDAVFTENY TKLRKQLAAK KYLNDLKKGG T-NH₂ | 31 | 6 |
| R3P3 | 1 HSDAVFTENY TKLRKQLAAK KYLNDLKKGG T | 31 | 8 |
| R3P12 | 1 HSDAVFTDNY TRLRKQLAAK KYLNDIKKGG T | 31 | 15 |
| R3P13 | 1 HSDAVFTDNY TRLRKQLAAK KYLNDIKK-NH₂ | 28 | 16 |
| R3P36 | 1 HSDAVFTDNY TRLRKQLAAK KYLNDIKKKR Y | 31 | 32 |
| R3P66 | 1 HSDAVFTDNY TRLRKQVAAK KYLQSIKNKR Y | 31 | 72 |

However, there is no teaching in the scientific or patent literature that suggests that select modifications to the VIP and PACAP sequences lead to a polypeptide with the ability to stimulate insulin secretion in a glucose-dependent fashion, and reduce plasma glucose concentration.

Certain terms used throughout this specification will now be defined, and others will be defined as introduced. The single letter abbreviation for a particular amino acid, its corresponding amino acid, and three letter abbreviation are as follows: A, alanine (ala); C, cysteine (cys); D, aspartic acid (asp); E, glutamic acid (glu); F, phenylalanine (phe); G, glycine (gly); H, histidine (his); I, isoleucine (ile); K, lycine (lys); L, leucine (leu); M, methionine (met); N, asparagine (asn); P, proline (pro); Q, glutamine (gln); R, arginine (arg); S, serine (ser); T, threonine (thr); V, valine (val); W, tryptophan (trp); Y, tyrosine (tyr).

The term "polynucleotide encoding a polypeptide" encompasses a polynucleotide which includes only coding sequence for the polypeptide as well as a polynucleotide which includes additional coding and/or non-coding sequence. The present invention further relates to polynucleotides which hybridize to the hereinabove-described sequences if there is at least about 70%, preferably at least about 90%, and more preferably at least about 95% identity between the sequences. The present invention particularly relates to polynucleotides encoding polypeptides which hybridize under stringent conditions to the hereinabove-described polynucleotides. As herein used, the term "stringent conditions" means "stringent hybridization conditions" Preferably, hybridization will occur only if there is at least about 90% and preferably about 95% through 97% identity between the sequences. The polynucleotides which hybridize to the hereinabove described polynucleotides in a preferred embodiment encode polypeptides which retain substantially the same biological function or activity as the mature polypeptide encoded by the cDNAs.

"Functional equivalent" and "substantially the same biological function or activity" each means that degree of biological activity that is within about 30% to 100% or more of that biological activity demonstrated by the polypeptide to which it is being compared when the biological activity of each polypeptide is determined by the same procedure. For example, a polypeptide that is functionally equivalent to a polypeptide of Fig. 1 is one that, when tested in the Cyclic AMP scintillation proximity assay of Specific Example 16, demonstrates accumulation of cAMP in CHO cell line expressing the human PACAP/VIP R2 (PACAP R3) receptor.

A polypeptide of this invention that is an R3 agonist is one that demonstrates about 30% -100% or more of maximal PACAP-27 R3 agonist activity when tested in the protocol of Example 16. The preferred polypeptides of this invention that are selective agonists for R3 over PACAP R2 and R1 receptors are those polypeptides that demonstrate the ratio of R3 agonist activity to R2 activity of about 10:1 or greater, and more preferably, about 100:1 or greater, and/or demonstrate the ratio of R3 agonist activity to R1 receptor activity of about 10:1 or greater, and more preferably, about 100:1 or greater when the polypeptide is tested in the protocol of Example 16, using cells that express the appropriate receptors.

"Stringent hybridization conditions" refers to an overnight incubation of the two pieces of polynucleotides to be hybridized at 42° C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C.

The terms "fragment," "derivative", and "variant", when referring to the polypeptides of FIG. 1, means fragments, derivatives, and variants of the polypeptides which retain substantially the same biological function or activity as such polypeptides, as described further below.

An analog includes a propolypeptide which includes within it, the amino acid sequence of the polypeptide of this invention. The active polypeptide of this invention can be cleaved from the additional amino acids that complete the propolypeptide molecule by natural, *in vivo* processes or by procedures well known in the art such as by enzymatic or chemical cleavage. For example, the 28-amino acid native peptide VIP is naturally expressed as a much larger polypeptide which is then processed *in vivo* to release the 28-amino acid active mature peptide.

A fragment is a portion of the polypeptide which retains substantially similar functional activity, as shown in the *in vivo* models disclosed herein as described further below.

A derivative includes all modifications to the polypeptide which substantially preserve the functions disclosed herein and include additional structure and attendant function, e.g., PEGylated polypeptides which have greater half-life, fusion polypeptides which confer targeting specificity or an additional activity such as toxicity to an intended target, as described further below.

The polypeptides of the present invention may be recombinant polypeptides, natural purified polypeptides or synthetic polypeptides.

The fragment, derivative, or variant of the polypeptides of the present invention may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethyleneglycol), or (iv) one in which the additional amino acids are fused to the mature polypeptide, such as a leader or secretory sequence or a sequence which is employed for purification of the mature polypeptide or a propolypeptide sequence, or v) one in which the polypeptide sequence is fused with a larger polypeptide, i.e. human albumin, a antibody or Fc, for increased duration of effect. Such fragments, derivatives, and variants and analogs are deemed to be within the scope of those skilled in the art from the teachings herein.

Preferably, the derivatives of the present invention will contain conservative amino acid substitutions (defined further below) made at one or more predicted, preferably nonessential amino acid residues. A "nonessential" amino acid residue is a residue that can be altered from the wild-type sequence of a protein without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Non conservative substitutions would not be made for conserved amino acid residues or for amino acid residues residing within a conserved protein domain, such as residues 19 and 27 where such residues are essential for protein activity such as R3 activity and/or R3 selectivity. Fragments, or biologically active portions include polypeptide fragments suitable for use as a medicament, to generate antibodies, as a research reagent, and the like. Fragments include peptides comprising amino acid sequences sufficiently similar to or derived from the amino acid sequences of a polypeptide of this invention and exhibiting at least one activity of that polypeptide, but which include fewer amino acids than the full-length polypeptides disclosed herein. Typically, biologically active portions comprise a domain or motif with at least one activity of the polypeptides. A biologically active portion of a polypeptide can be a peptide which is, for example, 5 or more amino acids in length. Such biologically active portions can be prepared synthetically or by recombinant techniques and can be evaluated for one or more of the functional activities of a polypeptide of this invention by means disclosed herein and/or well known in the art.

Moreover, preferred derivatives of the present invention include mature polypeptides that have been fused with another compound, such as a compound to increase the half-life of the polypeptide and/or to reduce potential immunogenicity of the polypeptide (for example, polyethyleneglycol "PEG"). In the case of PEGylation, the fusion of the polypeptide to PEG can be accomplished by any means known to one skilled in the art. For example, PEGylation can be accomplished by first introducing a cysteine mutation into the polypeptide, followed by site-specific derivatization with PEG-maleimide. The cysteine can be added to the C-terminus of the peptides. (See, for instance, Tsutsumi et al., Proc Natl Acad Sci U S A 2000 Jul 18:97(15):8548-53).

Variants of the polypeptides of this invention and polypeptides which are not part of the present invention include polypeptides having an amino acid sequence sufficiently similar to the amino acid sequence of the SEQ ID Nos of Fig. 1 or a domain thereof. The term "sufficiently similar" means a first amino acid sequence that contains a sufficient or minimum number of identical or equivalent amino acid residues relative to a second amino acid sequence such that the first and second amino acid sequences have a common structural domain and/or common functional activity. For example, amino acid sequences that contain a common structural domain that is at least 45%, preferably about 75% through 98%, identical are defined herein as sufficiently similar. Preferably, variants will be sufficiently similar to the amino acid sequence of the preferred polypeptides of this invention. Variants include variants of polypeptides encoded by a polynucleotide that hybridises to a polynucleotide of this invention or a complement thereof under stringent conditions. Such variants generally retain the functional activity of the polypeptides of this invention. Libraries of fragments of the polynucleotides can be used to generate a variegated population of fragments for screening and subsequent selection. For example, a library of fragments can be generated by treating a double-stranded PCR fragment of a polynucleotide with a nuclease under conditions wherein nicking occurs only about once per molecule, denaturing the double-stranded DNA, renaturing the DNA to form double-stranded DNA which can include sense/antisense pairs from different nicked products, removing single-stranded portions from reformed duplexes by treatment with S1 nuclease, and ligating the resulting fragment library into an expression vector. By this method, one can derive an expression library that encodes N-terminal and internal fragments of various sizes of the polypeptide of this invention.

Variants include polypeptides that differ in amino acid sequence due to mutagenesis. Variants that function as R3 agonists can be identified by screening combinatorial libraries of mutants, for example truncation mutants, of the polypeptides of this invention for R3 agonist activity.

In one embodiment, a variegated library of analogs is generated by combinatorial mutagenesis at the nucleic acid level and is encoded by a variegated gene library. A variegated library of variants can be produced by, for example, enzymatically ligating a mixture of synthetic oligonucleotides into gene sequences such that a degenerate set of potential variant amino acid sequences is expressible as individual polypeptides, or, alternatively, as a set of larger fusion proteins (for example, for phage display) containing the set of sequences therein. There are a variety of methods that can be used to produce libraries of potential variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential variant sequences. Methods for synthesizing degenerate oligonucleotides are known in the art (*see*, *e*.*g*., Narang (1983)Tetrahedron 39:3; Itakura et al (1984) Annu. Rev. Biochem. 53:323; Itakura et al (1984) Science 198:1056; Ike et al (1983) Nucleic Acid Res. 11:477).

Several techniques are known in the art for screening gene products of combinatorial libraries made by point mutations or truncation and for screening cDNA libraries for gene products having a selected property. Such techniques are adaptable for rapid screening of the gene libraries generated by the combinatorial mutagenesis of R-agonist polypeptides. The most widely used techniques, which are amenable to high through-put analysis for screening large gene libraries typically include cloning the gene library into replicable expression vectors, transforming appropriate cells with the resulting library of vectors and expressing the combinatorial genes under conditions in which detection of a desired activity facilitates isolation of the vector encoding the gene whose product was detected. Recursive ensemble mutagenesis (REM), a technique that enhances the frequency of functional mutants in the libraries, can be used in combination with the screening assays to identify the desired variants.

The invention also provides chimeric or fusion polypeptides. Examples include those polypeptides and polypeptides which are not part of the invention described in SEQ ID NOs 18 and 172 which are fusions of the pancreatic targeting sequence "SWCEPGWCR" (Rajotte D., et al ('998) J Clin Invest 102:430-437) with SEQ ID NOs 8 and 32, respectively. The targeting sequence is designed to localize the delivery of the polypeptide to the pancreas to minimize potential side effects. The polypeptides of this invention can be composed of amino acids joined to each other by peptide bonds or modified peptide bonds, i.e., peptide isosteres, and may contain amino acids other than the 20 gene-encoded amino acids. The polypeptides may be modified by either natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched, for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid deriative, covalent attachment of phosphotidylinositol, cross-linking cyclization, disulfide bond formation, demethylation, formation of covalent cross-links, formation of cysteine, formation of pyroglutamate, formulation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, Proteins, Structure and Molecular Properties, 2nd ed., T.E. Creighton, W.H. Freeman and Company, New York (1993); Posttranslational Covalent Modification of Proteins, B.C. Johnson, ed., Academic Press, New York, pgs 1-12 (1983); Seifter et al., Meth. Enzymol 182:626-646 (1990); Rattan et al., Ann. N.Y. Acad. Sci. 663:48-62 (1992)).

The polypeptides of the present invention and polypeptides which are not part of the present invention include the polypeptides of Fig. 1 that are SEQ ID NOs: 11 through 14, SEQ ID NO: 18, SEQ ID NOs: 21 through 26, SEO ID NOs: 32 through 36, SEQ ID NOs: 40 through 53, SEQ ID NOs: 57 through 61, SEQ ID NOs: 63 through 99, SEQ ID NOs: 102 through 119, SEO ID NOs: 121 through 137. SEQ ID NOs: 139 through 177, SEQ ID NOs: 179, 180, SEQ ID NOs: 183 through 202, 322 through 341, as well as those sequences having insubstantial variations in sequence from them. An "insubstantial variation" would include any sequence, substitution, or deletion variant that maintains substantially at least one biological function of the polypeptides of this invention, preferably R3 agonist activity, and more preferably selective R3 agonist activity, and most preferably, the insulin secreting activity demonstrated herein. These functional equivalents may preferably include polypeptides which have at least about a 90% identity to the polypeptides of Fig. 1, and more preferably at least a 95% identity to the polypeptides of Fig. 1, and still more preferably at least a 97% identity to the polypeptides of Fig.1, and also include portions of such polypeptides having substantially the same biological activity. However, any polypeptide having insubstantial variation in amino acid sequence from the polypeptides of Fig. 1 that demonstrates functional equivalency as described further herein is included in the description of the present invention.

As known in the art "similarity" between two polypeptides is determined by comparing the amino acid sequence and its conserved amino acid substitutes of one polypeptide to the sequence of a second polypeptide. Such conservative substitutions include those described above and by Dayhoff in The Atlas of Protein Sequence and Structure 5 (1978), and by Argos in EMBO J., 8:779-785 (1989). For example, amino acids belonging to one of the following groups represent conservative changes:
- ala, pro, gly, gin, asn, ser, thr;
- cys, ser, tyr, thr;
- val, ile, leu, met, ala, phe;
- lys, arg, his;
- phe, tyr, trp, his; and
- asp, glu.

The present invention also relates to vectors which include polynucleotides of the present invention, host cells which are genetically engineered with vectors of the invention and the production of polypeptides of the invention by recombinant techniques. Host cells may be genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, or selecting transformants. The culture conditions, such as temperature, pH and the like, are those previously used with the host cell selected for expression, and will be apparent to the ordinarily skilled artisan. The polynucleotide of the present invention may be employed for producing a polypeptide by recombinant techniques. Thus, for example, the polynucleotide sequence may be included in any one of a variety of expression vehicles, in particular vectors or plasmids for expressing a polypeptide. Such vectors include chromosomal, non-chromosomal and synthetic DNA sequences, e.g., derivatives of SV40; bacterial plasmids; phage DNA; yeast plasmids; vectors derived from combinations of plasmids and phage DNA, viral DNA such as vaccinia, adenovirus, fowl pox virus, and pseudorables. However, any other vector or plasmid may be used as long as they are replicable and viable in the host.

The appropriate DNA sequence may be inserted into the vector by a variety of procedures. In general, the DNA sequence is Inserted Into an appropriate restriction endonuclease site by procedures known in the art. Such procedures and others are deemed to be within the scope of those skilled in the art. The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda P_{L} promoter and other promoters known to control expression of genes in prokaryotic or eukaryotic cells or their viruses. The expression vector also contains a ribosome binding site for translation initiation and a transcription terminator. The vector may also Include appropriate sequences for amplifying expression. In addition, the expression vectors preferably contain a gene to provide a phenotypic trait for selection of transformed host cells such as dihydrofolate reductase or neomycin resistance for eukaryotic cell culture, or such as tetracycline or ampicillin resistance in E. coli. The vector containing the appropriate DNA sequence as herein above described, as well as an appropriate promoter or control sequence, may be employed to transform an appropriate host to permit the host to express the protein. As representative examples of appropriate hosts, there may be mentioned: bacterial cells, such as E. coli, Salmonella typhimurium. Streptomyces; fungal cells, such as yeast; insect cells, such as Drosophila S2 and Spodoptera Sf9; animal cells such as CHO, COS or Bowes melanoma; adenoviruses; plant cells, etc. The selection of an appropriate host is deemed to be within the scope of those skilled in the art from the teachings herein.

The present invention also includes recombinant constructs comprising one or more of the sequences of the invention as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example. Bacterial: pQE70, pQE60, pQE-9 (Qiagen), pBS, phagescript, psiX174, pBluescript SK, pBsKS, pNH8a, pNH16a, pNH18a, pNH46a (Stratagene); pTRC99A, pKK223-3, pKK233-3, pDR540, PRIT5 (Pharmacia). Eukaryotic: pWLneo, pSV2cat, pOG44, pXT1, pSG (Stratagene) pSVK3, pBPV, pMSG, PSVL (Pharmacia). However, any other plasmid or vector may be used as long as they are replicable and viable in the host. Promoter regions can be selected from any desired gene using CAT(chioramphenicol transferase) vectors or other vectors with selectable markers. Two appropriate vectors are pKK232-8 and pCM7. Particular named bacterial promoters Include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

The present invention also relates to host cells containing the above-described construct. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation (Davis, L., Dibner, M., Battey, I., Basic Methods in Molecular Biology, (1986)). The constructs in host cells can be used in a conventional manner to produce the gene product encoded by the recombinant sequence. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, (Cold Spring Harbor, N.Y., 1989), the disclosure of which is hereby incorporated by reference.

Transcription of a DNA encoding the polypeptides of the present invention by higher eukaryotes is increased by inserting an enhancer sequence into the vector. Enhancers are cis-acting elements of DNA, usually from about 10 to 300 bp, that act on a promoter to increase its transcription. Examples include the SV40 enhancer on the late side of the replication origin (bp 100 to 270), a cytomegalovirus early promoter enhancer, a polyoma enhancer on the late side of the replication origin, and adenovirus enhancers. Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), α factor, acid phosphatase, or heat shock proteins, among others. The heterologous structural sequence is assembled in appropriate phase with translation, initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, e.g., stabilization or simplified purification of expressed recombinant product.

Useful expression vectors for bacterial use are constructed by inserting a structural DNA sequence encoding a desired protein together with suitable translation, initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E.coli, Bacillus subtilis, Salmonella typhimurium and various species within the genera Pseudomonas, Streptomyces, and Staphylococcus, although others may also be employed as a matter of choice. Useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, Wis., USA). These pBR322 "backbone" sections are combined with an appropriate promoter and the structural sequence to be expressed.

After transformation of a suitable host strain and growth of the host strain to an appropriate cell density, the selected promoter is derepressed by appropriate means (e.g., temperature shift or chemical induction) and cells are cultured for an additional period. Cells are typically harvested by centrifugation, disrupted by physical or chemical means, and the resulting crude extract retained for further purification. Microbial cells employed in expression of proteins can be disrupted by any convenient method, including freeze-thaw cycling, sonication, mechanical disruption, or use of cell lysing agents.

Various mammalian cell culture systems can also be employed to express recombinant protein. Examples of mammalian expression systems include the COS-7 lines of monkey kidney fibroblasts, described by Gluzman, Cell 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 viral genome, for example, SV40 origin, early promoter, enhancer, splice, and polyadenylation sites may be used to provide the required non-transcribed genetic elements.

The polypeptides of the present invention may be recovered and purified from recombinant cell cultures by methods used heretofore, including ammonium sulfate or ethanol precipitation, acid extraction, anion or cation exchange chromatography, phosphocellulose chromatography, hydrophobic interaction chromatography, affinity chromatography, hydroxyapatite chromatography and lectin chromatography. Protein refolding steps can be used, as necessary, in completing configuration of the mature protein. Finally, high performance liquid chromatography (HPLC) can be employed for final purification steps.

The polypeptides of this invention may be a product of chemical synthetic-procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of this invention may be glycosylated with mammalian or other eukaryotic carbohydrates or may be non-glycosylated. Polypeptides of this invention may also include an initial methionine amino acid residue. An isolated or purified polypeptide of this invention, or biologically active portion thereof, is substantially free of other cellular material, or culture medium when produced by recombinant techniques, or substantially free of chemical precursors or other chemicals when chemically synthesized. Preferably, an isolated polypeptide of this invention is substantially free of cellular material and has less than about 30% (by dry weight) of non-polypeptide, or contaminating, material. When the polypeptide of this invention or a biologically active portion thereof is recombinantly produced, preferably culture medium represents less than about 30% of the volume of the polypeptide preparation. When this invention is produced by chemical synthesis, preferably the preparations contain less than about 30% by dry weight of chemical precursors or non-invention chemicals.

The polypeptides of this invention can be conveniently isolated as described in the specific examples below. A preparation of purified polypeptide is at least about 70% pure; preferably, the preparations are 85% through 99% pure. Purity of the preparations can be assessed by any means known in the art, such as SDS-polyacrylamide gel electrophoresis and Mass Spec/Liquid Chromatography.

Polynucleotide sequences encoding a polypeptide of this invention can be synthesized, in whole or in part, using chemical methods well known in the art (see, for example, Caruthers et al, Nucl. Acids Res. Symp. Ser. 215 - 223, 1980; Horn et al, Nucl. Acids Res. Symp. Ser 225-232, 1980). The polynucleotide that encodes the polypeptide can then be cloned into an expression vector to express the polypeptide.

As will be understood by those of skill in the art, it may be advantageous to produce the polypeptide-encoding nucleotide sequences possessing non-naturally occurring codons. For example, codons preferred by a particular prokaryotic or eukaryotic host can be selected to increase the rate of polypeptide expression or to produce an RNA transcript having desirable properties, such as a half-life which is longer than that of a transcript generated from the naturally occurring sequence.

The nucleotide sequences disclosed herein can be engineered using methods generally known in the art to alter the polypeptide-encoding sequences for a variety of reasons, including but not limited to, alterations which modify the closing, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

Alternatively, the polypeptides of this invention can be produced using chemical methods to synthesize its amino acid sequence, such as by direct peptide synthesis using solid-phase techniques (see, for example, Merrifield, J. Am. Chem. Soc. 85, 2149 - 2154, 1963; Roberge et al, Science, 269, 202 - 204, 1995). Polypeptide synthesis can be performed using manual techniques or by automation. Automated synthesis can be achieved, for example, using Applied Biosystems 431A Peptide Synthesizer (Perkin Elmer). Optionally, fragments of the polypeptide can be separately synthesized and combined using chemical methods to produce a full-length molecule.

The newly synthesized polypeptide can be substantially purified by preparative high performance liquid chromatography (see, for example, Creighton, PROTEINS: STRUCTURES AND MOLECULAR PRINCILPLES, WH Freeman and Co., New York, N.Y., 1983). The composition of a synthetic polypeptide of the present invention can be confirmed by amino acid analysis or sequencing by, for example, the Edman degradation procedure (see, Creighton, *supra*). Additionally, any portion of the amino acid sequence of the polypeptide can be altered during direct synthesis and/or combined using chemical methods with sequences from other proteins to produce a variant polypeptide or a fusion polypeptide.

The polypeptides of the present invention, as a result of the ability to stimulate insulin secretion from pancreatic islet cells *in vitro,* and by causing a decrease in blood glucose *in vivo*, may be employed in treatment of type 2 Diabetes (non-insulin dependent diabetes mellitus). Also, the polypeptides may be used to prevent subjects with impaired glucose tolerance from proceeding to develop type 2 diabetes. In addition, the polypeptides of the invention may be used for treatment of asthma (Bolin et al Biopolymer 37:57-66 (1995); US Patent No. 5,677,419)(showing that polypeptide R3P0 is active in relaxing guinea pig tracheal smooth muscle); hypotension induction (VIP induces hypotension, tachycardia, and facial flushing in asthmatic patients (Morice, A.H., and Sever, P.S., Peptides 7:279-280 (1986); Morice, A., et al., The Lancet II, 1225-1227 (1983)), male reproduction problems (Siow, Y., et al., Effects of vasoactive intestinal peptide on human sperm motility, Arch. Androl. 1999 Jul-Aug; 43(1):67-71); as a anti-apoptosis/neuroprotective agent (Brenneman D.E., et al., VIP neurotrophism in the central nervous system: multiple effectors and identification of a femtomolar-acting neuroprotective peptide, Ann. N. Y. Acad. Sci. 1998 Dec. 11;865:207-12); cardioprotection during ischemic events ( Kalfin R., et al., Protective role of intracoronary vasoactive intestinal peptide in ischemic and reperfused myocardium, J. Pharmacol. Exp. Ther. 1994 Feb;268(2):952-8; Das, D.K., et al., Coordinated role of vasoactive intestinal peptide and nitric oxide in cardioprotection, Ann. N. Y. Acad. Sci. 1998 Dec 11;865:297-308), and finally as an anti-ulcer agent (Tuncel, et al., The protective effect of vasoactive intestinal peptide (VIP) on stress-induced gastric ulceration in rats, Ann. N. Y. Acad. Sci. 1998 Dec 11;865:309-22.

The polypeptides of the present invention may be employed in combination with a suitable pharmaceutical carrier to comprise a pharmaceutical composition for parenteral administration. Such compositions comprise a therapeutically effective amount of the polypeptide and a pharmaceutically acceptable carrier or excipient. Such a carrier includes but is not limited to saline, buffered saline, dextrose, water, glycerol, ethanol, and combinations thereof. The formulation should suit the mode of administration. The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

The pharmaceutical compositions may be administered in a convenient manner such as by the oral, topical, intravenous, intraperitoneal, intramuscular, subcutaneous, intranasal or intradermal routes. The pharmaceutical compositions are administered in an amount which is effective for treating and/or prophylaxis of the specific indication. In general, they are administered in an amount of at least about 350ng (0.1nmol)/kg body weight and in most cases they will be administered in an amount not in excess of about 35ug (10nmol)/kg body weight per day. In most cases, the dosage is from about 0.1 µg/kg to about 100 mg/kg body weight daily, taking into account the routes of administration, symptoms, etc. These numbers do not take into account the bioavailability of the peptide in vivo, in which case more or less may be used to attain the effective dose desired. One of ordinary skill in the art is able to determine through dosing experiments or other conventional means the gross amount to be used to produce an effective dosage.

A polypeptide of the invention may also be employed in accordance with the present invention by expression of such polypeptide in vivo, which is often referred to as "gene therapy." Thus, for example, cells may be engineered with a polynucleotide (DNA or RNA) encoding for the polypeptide ex vivo, the engineered cells are then provided to a patient to be treated with the polypeptide. Such methods are well-known in the art. For example, cells may be engineered by procedures known in the art by use of a retroviral particle containing RNA encoding for the polypeptide of the present invention.

Local delivery of the insulin secretagogues using gene therapy may provide the therapeutic agent to the target area, i.e., the pancreas. For instance a pancreas-specific promoter was used to create a beta-cell pancreatic tumor mouse model (Hanahan, D., Heritable formation of pancreatic beta-cell tumors in transgenic mice expressing recombinant insulin/simian virus 40 oncogenes, Nature 315(6015):115-22 (1985)).

Both in vitro and in vivo gene therapy methodologies are contemplated. Several methods for transferring potentially therapeutic genes to defined cell populations are known. See, e.g., Mulligan, "The Basic Science Of Gene Therapy", Science, 260: 926-31 (1993). These methods include:
1) Direct gene transfer. See, e.g., Wolff et al., "Direct Gene transfer Into Mouse Muscle In Vivo", Science, 247:1465-68 (1990);
2) Liposome-mediated DNA transfer. See, e.g., Caplen at al., "Liposome-mediated CFTR Gene Transfer To The Nasal Epithelium Of Patients With Cystic Fibrosis", Nature Med. 3: 39-46 (1995); Crystal, "The Gene As A Drug", Nature Med. 1:15-17 (1995); Gao and Huang, "A Novel Cationic Liposome Reagent For Efficient Transfection Of Mammalian Cells", Biochem. Biophys. Res. Comm., 179:280-85 (1991);
3) Retrovirus-mediated DNA transfer. See, e.g., Kay et al., "In Vivo Gene Therapy Of Hemophilia B: Sustained Partial Correction In Factor IX-Deficient Dogs", Science, 262:117-19 (1993); Anderson, "Human Gene Therapy", Science, 256:808-13 (1992).
4) DNA Virus-mediated DNA transfer. Such DNA viruses include adenoviruses (preferably Ad-2 or Ad-5 based vectors), herpes viruses (preferably herpes simplex virus based vectors), and parvoviruses (preferably "defective" or non-autonomous parvovirus based vectors, more preferably adeno-associated virus based vectors, most preferably AAV-2 based vectors). See, e.g., Ali et al., "The Use Of DNA Viruses As Vectors For Gene Therapy", Gene Therapy, 1:367-84 (1994); United States Patent 4,797,368, incorporated herein by reference, and United States Patent 5,139,941, incorporated herein by reference.

The choice of a particular vector system for transferring the gene of interest will depend on a variety of factors. One important factor is the nature of the target cell population. Although retroviral vectors have been extensively studied and used in a number of gene therapy applications, these vectors are generally unsuited for infecting non-dividing cells. In addition, retroviruses have the potential for oncogenicity. However, recent developments in the field of lentiviral vectors may circumvent some of these limitations. See Naldini et al, In vivo gene delivery and stable transduction of nondividing cells by a lentiviral vector, Science 272:263-7 (1996).

Retroviruses from which the retroviral plasmid vectors hereinabove mentioned may be derived include, but are not limited to, Moloney Murine Leukemia Virus, spleen necrosis virus, retroviruses such as Rous Sarcoma Virus, Harvey Sarcoma Virus, avian leukosis virus, gibbon ape leukemia virus, human immunodeficiency virus, adenovirus, Myeloproliferative Sarcoma Virus, and mammary tumor virus. In one embodiment, the retroviral plasmid vector is derived from Moloney Murine Leukemia Virus.

Adenoviruses have the advantage that they have a broad host range, can infect quiescent or terminally differentiated cells, such as neurons or hepatocytes, and appear essentially non-oncogenic. See, e.g., Ali et al., supra, p. 367. Adenoviruses do not appear to integrate into the host genome. Because they exist extrachromosomally, the risk of insertional mutagenesis is greatly reduced. Ali et al., supra, p. 373.

Adeno-associated viruses exhibit similar advantages as adenoviral-based vectors. However, AAVs exhibit site-specific integration on human chromosome 19 (Ali et al., supra, p. 377).

In a preferred embodiment, the DNA encoding the polypeptide insulin secretagogues of this invention is used in gene therapy for disorders such as diabetes.

According to this embodiment, gene therapy with DNA encoding polypeptide insulin secretagogues or muteins of this invention is provided to a patient in need thereof, concurrent with, or immediately after diagnosis.

The skilled artisan will appreciate that any suitable gene therapy vector containing polypeptide insulin secretagogues, DNA or DNA of fragment, derivative or variant of polypeptide insulin secretagogues may be used in accordance with this embodiment. The techniques for constructing such a vector are known. See, e.g., Anderson, W.F., "Human Gene Therapy," Nature, 392 25-30 (1998); Verma, LM., and Somia, N., "Gene Therapy - Promises, Problems, and Prospects," Nature, 389 239-242 (1998). Introduction of the polypeptide insulin secretagogues DNA-containing vector to the target site may be accomplished using known techniques.

The vector includes one or more promoters. Suitable promoters which may be employed include, but are not limited to, the retroviral LTR; the SV40 promoter; and the human cytomegalovirus (CMV) promoter described in Miller, et al., Biotechniques, 7(9): 980-990 (1989), or any other promoter (e.g., cellular promoters such as eukaryotic cellular promoters including, but not limited to, the histone, pol III, and β-actin promoters). Other viral promoters which may be employed include, but are not limited to, adenovirus promoters, thymidine kinase (TK) promoters, and B19 parvovirus promoters. The selection of a suitable promoter will be apparent to those skilled in the art from the teachings contained herein.

The nucleic acid sequence encoding the polypeptide of the present invention is under the control of a suitable promoter. Suitable promoters which may be employed include, but are not limited to, adenoviral promoters, such as the adenoviral major late promoter; or hetorologous promoters, such as the cytomegalovirus (CMV) promoter; the respiratory syncytial virus (RSV) promoter; inducible promoters, such as the MMT promoter, the metallothionein promoter; heat shock promoters; the albumin promoter; the ApoAl promoter; human globin promoters; viral thymidinekinase promoters, such as the Herpes Simplex thymidine kinase promoter; retroviral LTRs (including the modified retroviral LTRs hereinabove described); the β-actin promoter; and human growth hormone promoters. The promoter also may be the native promoter which controls the gene encoding the polypeptide.

The retroviral plasmid vector is employed to transduce packaging cell lines to form producer cell lines. Examples of packaging cells which maybe transfected include, but are not limited to, the PE501, PA317, ψ-2, ψ-AM, PA12, T19-14X, VT-19-17-H2, ψCRE, ψCRIP, GP+E-86,GP+envAm12, and DAN cell lines as described in Miller, Human Gene Therapy, 1: 5-14 (1990), which is incorporated herein by reference in its entirety. The vector may transduce the packaging cells through any means known in the art. Such means include, but are not limited to, electroporation, the use of liposomes, and CaPO₄ precipitation. In one alternative, the retroviral plasmid vector may be encapsulated into a liposome, or coupled to a lipid, and then administered to a host. The producer cell line generates infectious retroviral vector particles which include the nucleic acid sequence(s) encoding the polypeptides. Such retroviral vector particles then may be employed, to transduce eukaryotic cells, either in vitro or in vivo. The transduced eukaryotic cells will express the nucleic acid sequence(s) encoding the polypeptide. Eukaryotic cells which may be transduced include, but are not limited to, non-human embryonic stem cells, embryonic carcinoma cells, as well as non-human hematopoietic stem cells, hepatocytes, fibroblasts, myoblasts, keratinocytes, endothelial cells, and bronchial epithelial cells.

A different approach to gene therapy is "transkaryotic therapy" wherein the patient's cells are treated ex vivo to induce the dormant chromosomal genes to produce the protein of interest after reintroduction to the patient. Transkaryotic therapy assumes the individual has a normal complement of genes necessary for activation. Transkaryotic therapy involves introducing a promoter or other exogenous regulatory sequence capable of activating the nascent genes, into the chromosomal DNA of the patients' cells ex vivo, culturing and selecting for active protein-producing cells, and then reintroducing the activated cells into the patient with the intent that they then become fully established. The "gene activated" cells then manufacture the protein of interest for some significant amount of time, perhaps for as long as the life of the patient. U.S. Patent Nos. 5,641,670 and 5,733,761 disclose in detail this concept, and are hereby incorporated by reference in their entirety.

In order that this invention may be better understood, the following examples are set forth. These examples are for the purpose of illustration only, and are not to be construed as limiting the scope of the invention in any manner. All publications mentioned herein are incorporated by reference in their entirety.

### Example 1. Protocol for Rat Islet Isolation

Sprague Dawley rats (275-320 g) were used as the source of donor islets. Briefly, the pancreas was filled with 10 ml of cold reconstituted Liberase RI (Boehringer Manheim), harvested and incubated with additional 5 ml enzyme solution in water bath for 30 minutes. Tissue suspension was washed twice with cold 10% FBS/Hanks buffer (Gibco), resuspended in 8 ml 25% ficoll (Sigma) and then layered with 5 ml each of 23%, 20% and 11 % ficoll. The islets in the 20% layer after centrifugation were removed, washed twice with cold 10% FBS/Hank buffer and resuspended in 10% FBS/RPMI 1640 media (Sigma).

### Example 2. Protocol for Assaying Peptide-Induced Elevation of Insulin Levels in Rats.

Wistar rats are fasted overnight (17 hrs) and then anesthetized with pentobarbital (0.1 ml/100 g BW). Glucose (0.4 g/kg dissolved in 1% human albumin-saline) +/- peptide (dissolved in 1% human albumin-saline) is injected intravenously into the tail vein.
The rats are eye-bled 1 minute after the injection and 50-100ul of the plasma are assayed for insulin level with the Linco RIA kit (Linco Research, Inc., St. Charles, MO).

### Example 3. Protocol for Determining the Effect of Peptides on Intraperitoneal Glucose Tolerance in Rats.

Wistar rats are fasted overnight and then anesthetized with pentobarbital. The rats were eye-bled (zero time) and the peptide ( in 1% human albumin) was injected into the tail vein. Five minutes later, 1 g/kg of glucose (in saline) was injected intraperitoneally, and the rats were eye-bled after 15, 30 and 60 minutes. Plasma glucose levels were determined using the Technicon Axon autoanalyzer, Bayer Diagnosics division of Bayer Corporation, Tarrytown NY, operated using Method No. SM4-2143F90 "Glucose".

### Example 4. Protocol for Determining the Effect of Peptides on Intestinal Water Retention in Rats.

Male rats were fasted for 24 hours, and their water bottles were taken away for 2 - 3 hours before the start of the experiment. Peptide or saline was injected subcutaneously into conscious rats. The rats were euthanized with CO₂ 10 minutes after dosing, and the small intestine dissected out and weighed (1). The intestine was cut open, the water in the lumen absorbed with filter paper, and the intestine reweighed (2). The amount of intestinal water (g) = weight (1) - weight (2).

### Example 5. Peptide Synthesis Methodology.

The following general procedure was followed to synthesize some of the polypeptides of the invention. Peptide synthesis was carried out by the FMOC/t-Butyl strategy (Peptide Synthesis Protocols (1994), Volume 35 by Michael W. Pennington & Ben M. Dunn) under continuous flow conditions using Rapp-Polymere PEG-Polystyrene resins (Rapp-Polymere, Tubingen, Germany). At the completion of synthesis, peptides are cleaved from the resin and de-protected using TFA/DTT/H₂O/Triisopropyl silane (88/5/5/2). Peptides were precipitated from the cleavage cocktail using cold diethyl ether. The precipitate was washed three times with the cold ether and then dissolved in 5% acetic acid prior to lyophilization. Peptides were checked by reversed phase chromatography on a YMC-Pack ODS-AQ column (YMC, Inc., Wilmington, NC) on a Waters ALLIANCE® system (Waters Corporation, Milford, MA) using water/acetonitrile with 3% TFA as a gradient from 0% to 100% acetonitrile, and by MALDI mass spectrometry on a VOYAGER DE^{™} MALDI Mass Spectrometer, (model 5-2386-00, PerSeptive BioSystems, Framingham, MA). Matrix buffer (50/50 dH2O/acetonitrile with 3% TFA) peptide sample added to Matrix buffer 1/1. Those peptides not meeting the purity criteria of >95% are purified by reversed phase chromatography on a Waters Delta Prep 4000 HPLC system (Waters Corporation, Milford, MA).

### Example 6. Peptide Cloning

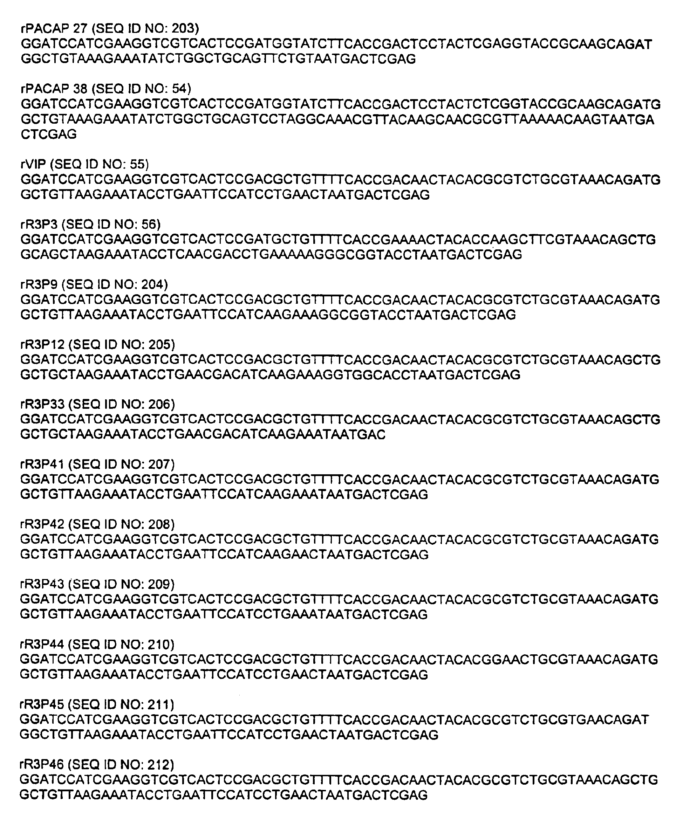

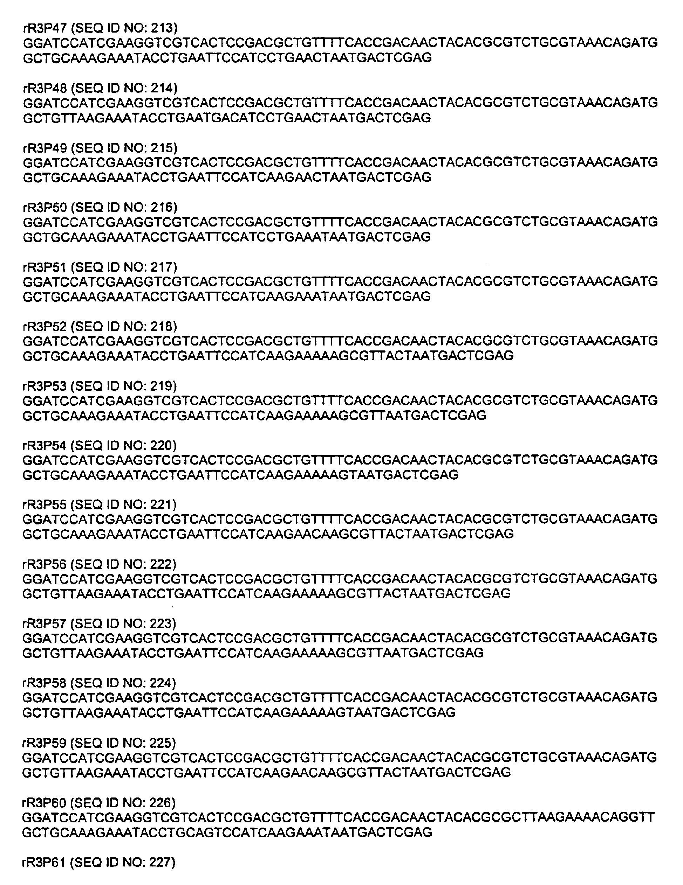

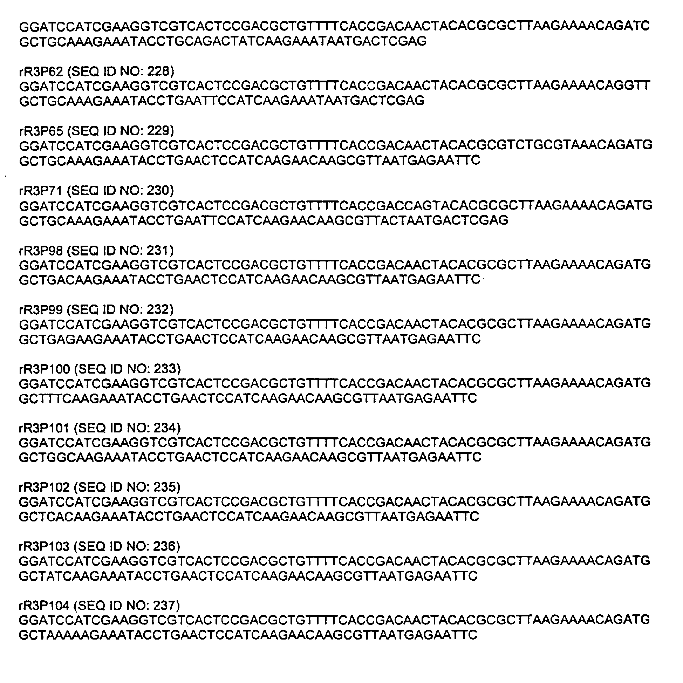

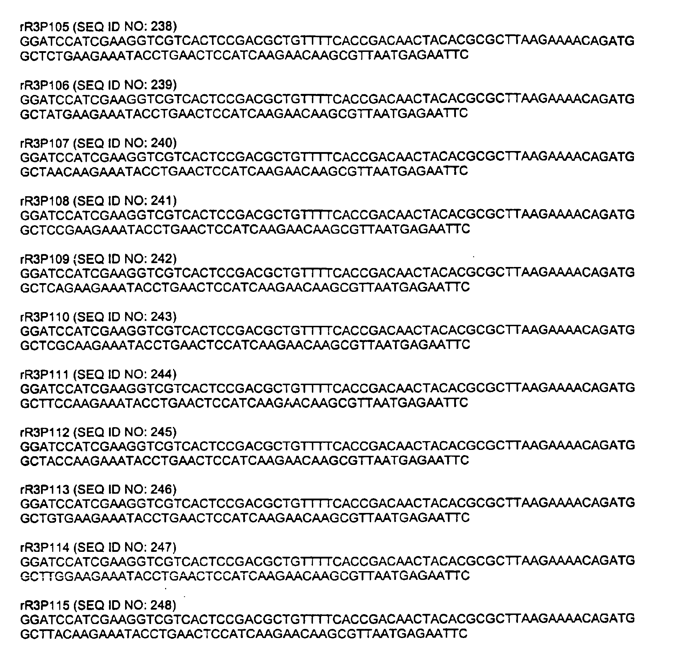

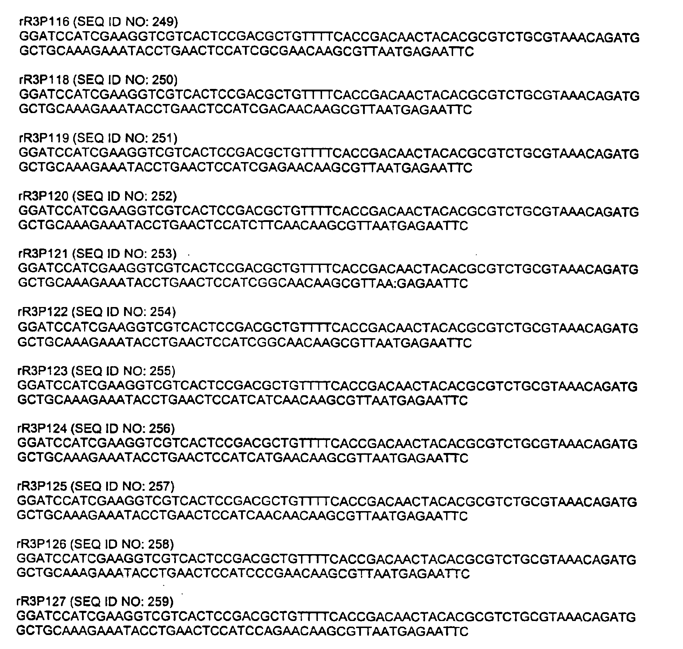

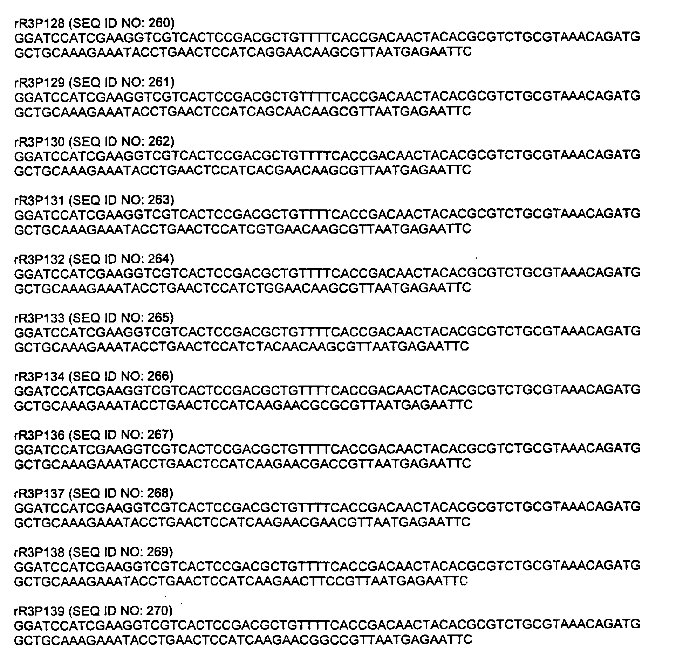

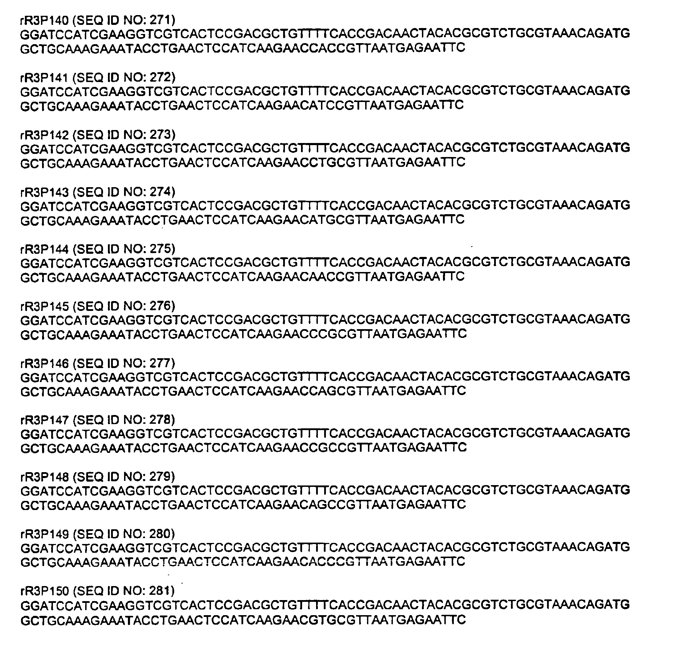

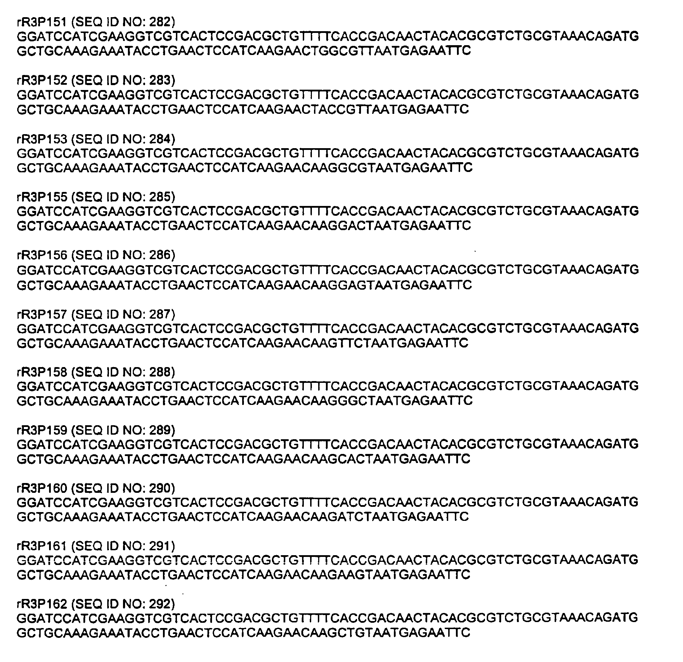

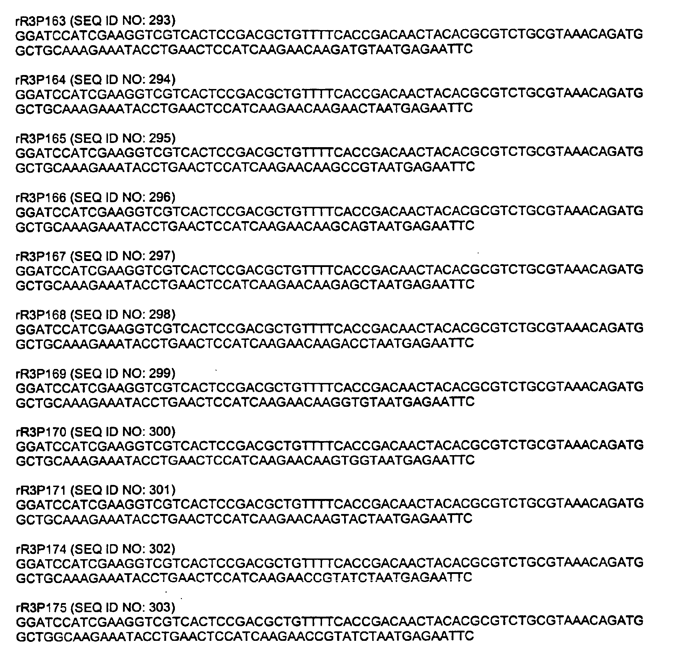

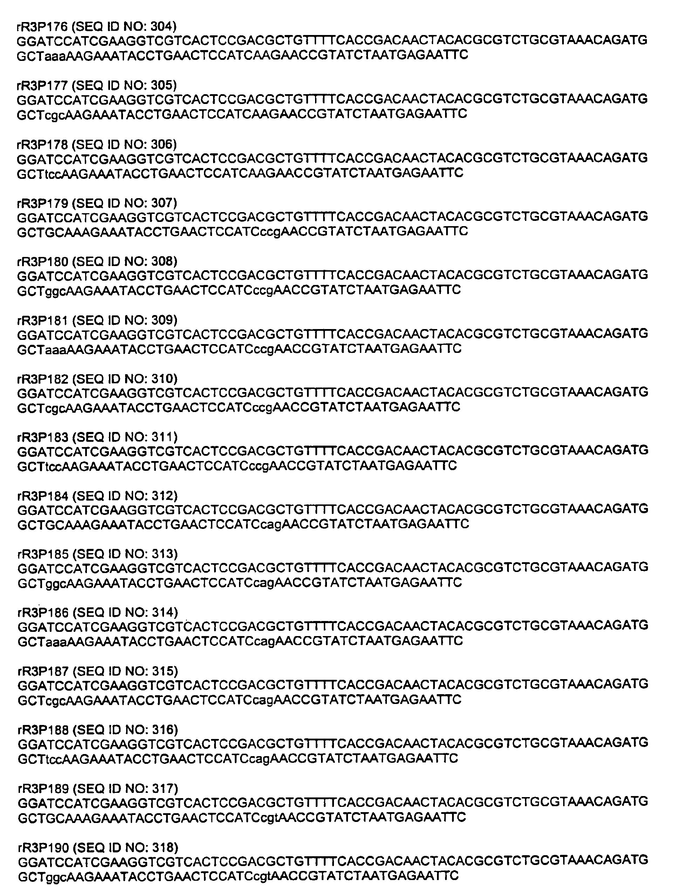

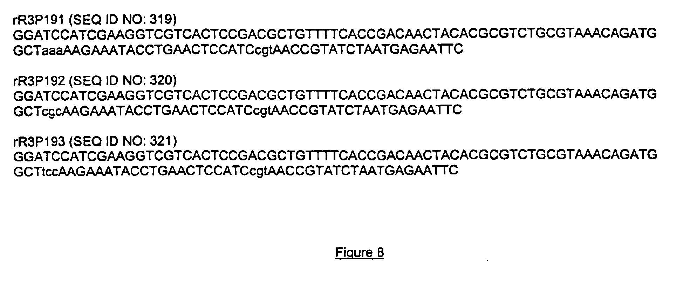

The recombinant expression of VIP has been attempted previously with mixed results. Simoncsits et al (Eur. J. Biochem. 178: 343-350, (1988)) expressed Leu¹⁷, Gly²⁹-VIP or Leu¹⁷Gly²⁹ Lys³⁰Arg³¹-VIP as a C-terminal fusion to the N-terminal part of the E. coli β-galactosidase gene in E. coli. The removal of the methionine at position 17 eliminates a CNBR cleavage site. C-terminal addition of Gly or Gly-Lys-Arg was designed for potential in vivo C-terminal amidation by mammalian PAMase. Upon CNBR cleavage of the fusion proteins at the methionine introduced N-terminal to the VIP mutants, free VIP mutants were purified and shown to possess similar activities as the native VIP, although the activities were measured only at saturating concentrations of the peptides. Raingeaud et al (Biochimie 78: 14-25 (1996)) expressed VIP as polymeric C-terminal fusions to glutathione S-transferase (GST) in E. coli. The free polymeric or monomeric VIP peptides were released upon sequential cleavages by Factor Xa and hydroxylamine. The requirement for the two-step cleavage led to inefficiency and a mixture of products. Polymeric or monomeric VIP peptides produced by this method were less active than the native VIP. An improved version of the construct using only Factor Xa cleavage yielded a VIP mutant with a seven residue C-terminal extension that was also less active than the native VIP (Ottavi et al, Biochimie 80: 289-293 (1998)). No expression of PACAP has been reported to date. To establish a robust method for expressing PACAP, VIP, and their mutants, their genetic codes were cloned C-terminal to GST with a single Factor Xa recognition site separating the monomeric peptide and GST. The gene encoding Factor Xa recognition site fused to DNA sequence of the peptide to be produced has been synthesized by hybridizing two overlapping single-stranded DNA fragments (70-90mers) containing a Bam HI or Xho I restriction enzyme site immediately 5' to the DNA sequence of the gene to be cloned, followed by DNA synthesis of the opposite strands via the large fragment of DNA polymerase I (Life Technologies, Inc., Gaithersburg, MD). The DNA sequence chosen for each gene was based on the reverse translation of the designed amino acid sequence of each peptide. In some cases, the gene encoding the peptide is generated by PCR mutagenesis (Picard, V, et al., Nucleic Acids Res 22: 2587-91 (1994); Sambrook, J., Fritsch, E. F., & Maniatis, T. (1989) Molecular Cloning: A Laboratory Manual, 2nd ed., Cold Spring Harbor Laboratory Press, New York) of a gene already made by the method described above. The double-stranded product is then digested by Bam HI and Xho I and ligated into pGEX-6P-1 (Amersham Pharmacia Blotech) which has also been cleaved by Bam HI and Xho I. The DNA sequences of the cloned peptide genes are listed in Figure 8.

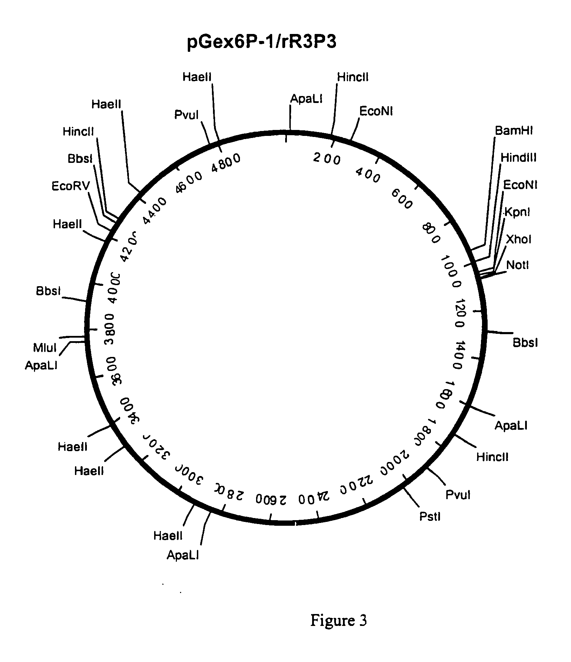

For example, when DNA sequences of SEQ ID NOs: 54, 55 and 56 (which are not polypeptide sequences provided by the invention) are cloned Into pGEX-6P-1, the following polypeptide sequences were expressed as fusions with glutathione S-transferase (GST):
PACAP38: IEGRHSDGIFTDSYSRYRKQMAVKKYLAAVLGKRYKQRVKNK (SEQ ID NO:2)
VIP: IEGRHSDAVFTDNYTRLRKQMAVKKYLNSILN (SEQ ID NO:1)
R3P3: IEGRHSDAVFTENYTKLRKQLAAKKYLNDLKKGGT (SEQ ID NO:8)
A restriction map of a typical plasmid containing the GST-peptide fusion is shown as Figure 3.

### Example 7. Peptide Recombinant Expression and Purification

BL21 cells (Stratagene) transformed with the GST-peptide fusion containing plasmids were grown at 37°C until OD₆₀₀ reached 0.6 to 1.0 and induced by 1 mM IPTG (Life Technologies) for 2 hours at 37°C. 2L of cells were spun at 7,700 g for 15 minutes, weighed, and stored at -20°C for at least 3 hours. The frozen cell pellet was resuspended in 100 mL ice-cold PBS with 250 µl of protease inhibitor cocktail (Cat No. P-8465, Sigma Chemical) per gram of cells, sonicated at 3x for 1 minute with 15 second breaks. Cellular debris were spun down at 10,000 g for 20 minutes. The supernatant was mixed with 2 mL of 50% Glutathione Sepharose 4B resin (Pharmacia) on a shaker overnight at 4 °C. The resins were spun down at 1,500 g for 15 minutes, packed into empty Poly-Prep Chromatography Columns (Bio-Rad), washed with 30 mL PBS followed by 10mL of Factor Xa buffer (1 mM CaCl₂, 100 mM NaCl, and 50 mM Tris-HCl, pH 8.0). The peptide were cleaved off the column by 60 units of Factor Xa (Pharmacia) in 1 mL of Factor Xa buffer for overnight at 4 °C and run on C18 HPLC (Beckman System Gold), using a 2 mL loop and flow rate of 2 mL/min with the following program; 10 minutes of Buffer A (0.1% TFA/H₂O), 30 minutes of gradient to Buffer B (0.1% TFA/ACN), 10 minutes of Buffer A, 10 minutes of gradient, and 10 minutes of Buffer A. Peak fractions (1 mL each) were collected and screened by 10-20% Tricine-SDS get electrophoresis. Fractions containing the peptides of Table 1 were pooled and dried down. Typical yields are several hundred micrograms of free peptides per liter of E. coli culture. Recombinant peptides have been shown to have the same activities as their synthetic versions.

The following table contains some selected polypeptide and polypeptides of sequences which are not part of the invention made according to the Peptide Synthesis protocol discussed above (example 5), or recombinantly as described in Example 7. Peptides produced by the recombinant method are denoted with a small letter "r" In front of the peptide designator. Peptides produced by both recombinant and synthetic means are noted by asterisks next to their Peptide No's. Peptides R3P0 and R3P4 were reported by Bolin, et al. (Biopolymers 37:57-66 (1995); US Patent 5,677,419).

**Table 1**

| **Peptide No.** | **Sequence** | **SEQ ID NO:** |
|---|---|---|
| R3P0 | Ac-HSDAVFTENYTKLRKQNIeAAKKYLNDLKKGGT-NH₂ | 5 |
| R3P1 | Ac-HSDAVFTENYTKLRKQLAAKKYLNDLKKGGT-NH₂ | 6 |
| R3P2 | Ac-HSDAVFTENYTKLRKQLAAKKYLNDLKKGGT | 7 |
| rR3P3* | HSDAVFTENYTKLRKQLAAKKYLNDLKKGGT | 8 |
| R3P4 | Ac-HSDAVFTEN(CH₃O-Y)TKLRKQNIeAAKKYLNDLKK-NH₂ | 9 |
| R3P5 | HSDAVFTENYTKLRKQLAAKKYLNDLKK | 10 |
| R3P8 | HSDAVFTDNYTRLRKQMAVKKYLNSIKK-NH₂ | 11 |
| rR3P9* | HSDAVFTDNYTRLRKQMAVKKYLNSIKKGGT | 12 |
| R3P10 | HSDAVFTENYTKLRKQLAAKKYLNDLLNGGT | 13 |
| R3P11 | HSDAVFTDNYTKLRKQLAAKKYLNDILNGGT | 14 |
| R3P12* | HSDAVFTDNYTRLRKQLAAKKYLNDIKKGGT | 15 |
| R3P13 | HSDAVFTDNYTRLRKQLAAKKYLNDIKK-NH₂ | 16 |
| R3P14 | HSDAVFTDNYTRLRKQMAVKKYLNDLKKGGT | 17 |
| R3P19 | HSDAVFTENYTKLRKQLAAKKYLNDLKKGGTSWCEPGWCR | 18 |
| R3P20 | HSDAVFTDNYTRLRKQMAAKKYLNDIKKGGT | 19 |
| R3P21 | HSDAVFTDNYTRLRKQLAVKKYLNDIKKGGT | 20 |
| R3P22 | HSDAVFTDNYTRLRKQLAAKKYLNSIKKGGT | 21 |
| R3P24 | HSDAVFTDNYTRLRKQLAAKKYLNDIKNGGT | 22 |
| R3P25 | HSDAVFTDNYTRLRKQLAVKKYLNSIKKGGT | 23 |
| R3P26 | HSDAVFTDNYTRLRKQMAAKKYLNSIKKGGT | 24 |
| R3P29 | HSDAVFTDNYTRLRKQLAVKKYLNDIKNGGT | 25 |
| R3P30 | HSDAVFTDNYTRLRKQLAAKKYLNSIKNGGT | 26 |
| R3P31 | HSDAVFTDNYTRLRKQLAAKKYLNDIKKGG | 27 |
| R3P32 | HSDAVFTDNYTRLRKQLAAKKYLNDIKKG | 28 |
| rR3P33* | HSDAVFTDNYTRLRKQLAAKKYLNDIKK | 29 |
| R3P34 | HSDAVFTDNYTRLRKQLAAKKYLNDIKKQ | 30 |
| R3P35 | HSDAVFTDNYTRLRKQLAAKKYLNDIKKNQ | 31 |
| R3P36 | HSDAVFTDNYTRLRKQLAAKKYLNDIKKKRY | 32 |
| rR3P41 | HSDAVFTDNYTRLRKQMAVKKYLNSIKK | 33 |
| rR3P42 | HSDAVFTDNYTRLRKQMAVKKYLNSIKN | 34 |
| rR3P43 | HSDAVFTDNYTRLRKQMAVKKYLNSILK | 35 |
| rR3P44 | HSDAVFTDNYTELRKQMAVKKYLNSILN | 36 |
| rR3P45 | HSDAVFTDNYTRLREQMAVKKYLNSILN | 37 |
| rR3P46 | HSDAVFTDNYTRLRKQLAVKKYLNSILN | 38 |
| rR3P47 | HSDAVFTDNYTRLRKQMAAKKYLNSILN | 39 |
| rR3P48 | HSDAVFTDNYTRLRKQMAVKKYLNDILN | 40 |
| rR3P49 | HSDAVFTDNYTRLRKQMAAKKYLNSIKN | 41 |
| rR3P50 | HSDAVFTDNYTRLRKQMAAKKYLNSILK | 42 |
| rR3P51* | HSDAVFTDNYTRLRKQMAAKKYLNSIKK | 43 |
| rR3P52 | HSDAVFTDNYTRLRKQMAAKKYLNSIKKKRY | 44 |
| rR3P53* | HSDAVFTDNYTRLRKQMAAKKYLNSIKKKR | 45 |
| rR3P54 | HSDAVFTDNYTRLRKQMAAKKYLNSIKKK | 46 |
| rR3P55* | HSDAVFTDNYTRLRKQMAAKKYLNSIKNKRY | 47 |
| rR3P56 | HSDAVFTDNYTRLRKQMAVKKYLNSIKKKRY | 48 |
| rR3P57 | HSDAVFTDNYTRLRKQMAVKKYLNSIKKKR | 49 |
| rR3P58 | HSDAVFTDNYTRLRKQMAVKKYLNSIKKK | 50 |
| rR3P59 | HSDAVFTDNYTRLRKQMAVKKYLNSIKNKRY | 51 |
| rR3P60 | HSDAVFTDNYTRLRKQVAAKKYLQSIKK | 52 |
| rR3P61 | HSDAVFTDNYTRLRKQIAAKKYLQTIKK | 53 |
| R3P6 | HSDGIFTESYSRYRKQMAVKKYLAALKKKRYKQRVKNK | 57 |
| R3P7 | HSDAVFTENYTRLRKQMAVKKYLNSLKK-NH₂ | 58 |
| R3P15 | HSDGIFTDSYSRYRKQMAVKKYLSAVRHGQT-NH₂ | 59 |
| R3P16 | HSDGIFTDSYSRYRKQMAVKKYLAAVKQGGT-NH₂ | 60 |
| R3P17 | HSDGIFTDSYSRYRKQMAVKKYLAAVKKYLAAVRHG-NH₂ | 61 |
| R3P18 | SWCEPGWCRHSDAVFTENYTKLRKQLAAKKYLNDLKKGGT | 62 |
| R3P23 | HSDAVFTDNYTRLRKQLAAKKYLNDILKGGT | 63 |
| R3P27 | HSDAVFTDNYTRLRKQLAAKKYLNDILNGGT | 64 |
| R3P28 | HSDAVFTDNYTRLRKQLAVKKYLNDILKGGT | 65 |
| R3P37 | HSDGIFTDSYSRYRKQLAAKKYLADVKKGGT | 66 |
| R3P38 | HSDGIFTDSYSRYRKQLAAKKYLADVKK | 67 |
| R3P39 | HSDGIFTDSYSRYRKQLAVKKYLAAVKK | 68 |
| R3P40 | HSDGIFTDSYSRYRKQMAVKKYLAAVKK | 69 |
| R3P62 | HSDAVFTDNYTRLRKQVAAKKYLNSIKK | 70 |
| R3P65 | HSDAVFTDNYTRLRKQMAAKKYLNSIKNKR | 71 |
| R3P66 | HSDAVFTDNYTRLRKQVAAKKYLQSIKNKRY | 72 |
| R3P67 | HSDAVFTDNYTRLRKQLAAKKYLNTIKNKRY | 73 |
| R3P68 | HSDAVFTDNYTRLRKQVAAKKYLNSIKNKRY | 74 |
| R3P69 | HSDAVFTDNYTRLRKQMAAKKYLQSIKNKRY | 75 |
| R3P70 | HSDAVFTDNYTRLRKQMAAKKYLNTIKNKRY | 76 |
| R3P71 | HSDAVFTDQYTRLRKQMAAKKYLNSIKNKRY | 77 |
| R3P72 | HSDAVFTDQYTRLRKQLAAKKYLNTIKNKRY | 78 |
| R3P73 | HSDAVFTDNYTRLRKQMAAHKYLNSIKNKRY | 79 |
| R3P74 | HSDAVFTDNYTRLRKQMAAKHYLNSIKNKRY | 80 |
| R3P75 | HSDAVFTDQYTRLRKQLAAHKYLNTIKNKRY | 81 |
| R3P76 | HSDAVFTDQYTRLRKQLAAKHYLNTIKNKRY | 82 |
| R3P77 | HSDAVFTDNYTRLRKQVAAKKYLQSIKKKR | 83 |
| R3P78 | HSDAVFTDNYTRLRKQVAAKKYLNSIKKKR | 84 |
| R3P79 | HSDAVFTDNYTRLRKQVAAKKYLNSIKNKRY | 85 |
| R3P80 | HSDAVFTDNYTRLRKQVAVKKYLQSIKKKR | 86 |
| R3P81 | HSDAVFTDNYTRLRKQVAVKKYLQSIKKK | 87 |
| R3P82 | HSDAVFTDNYTRLRKQVAVKKYLQSIKNKRY | 88 |
| R3P83 | HSDAVFTDNYTRLRKQVAAKKYLQSILKKRY | 89 |
| R3P84 | HSDAVFTDNYTRLRKQVAAKKYLQSILKKR | 90 |
| R3P85 | HSDAVFTDNYTRLRKQVAAKKYLQSILKK | 91 |
| R3P86 | HSDAVFTDNYTRLRKQVAAKKYLQSIKNK | 92 |
| R3P87 | HSDAVFTDNYTRLRKQVAVKKYLQSILKKRY | 93 |
| R3P88 | HSDAVFTDNYTRLRKQVAVKKYLQSILKKR | 94 |
| R3P89 | HSDAVFTDNYTRLRKQVAVKKYLQSILKK | 95 |
| R3P92 | HSDAVFTDNYTRLRKQVAAKKYLQSILNKRY | 97 |
| R3P93 | HSDAVFTDNYTRLRKQVAAKKYLQSILNKR | 98 |
| R3P94 | HSDAVFTDNYTRLRKQVAAKKYLQSILNK | 99 |
| rR3P97 | HSDAVFTDNYTRLRKQMACKKYLNSIKNKR | 100 |
| rR3P98 | HSDAVFTDNYTRLRKQMADKKYLNSIKNKR | 101 |
| rR3P99 | HSDAVFTDNYTRLRKQMAEKKYLNSIKNKR | 102 |
| rR3P100 | HSDAVFTDNYTRLRKQMAFKKYLNSIKNKR | 103 |
| rR3P101 | HSDAVFTDNYTRLRKQMAGKKYLNSIKNKR | 104 |
| rR3P102 | HSDAVFTDNYTRLRKQMAHKKYLNSIKNKR | 105 |
| rR3P103 | HSDAVFTDNYTRLRKQMAIKKYLNSIKNKR | 106 |
| rR3P104 | HSDAVFTDNYTRLRKQMAKKKYLNSIKNKR | 107 |
| rR3P105 | HSDAVFTDNYTRLRKQMALKKYLNSIKNKR | 108 |
| rR3P106 | HSDAVFTDNYTRLRKQMAMKKYLNSIKNKR | 109 |
| rR3P107 | HSDAVFTDNYTRLRKQMANKKYLNSIKNKR | 110 |
| rR3P108 | HSDAVFTDNYTRLRKQMAPKKYLNSIKNKR | 111 |
| rR3P109 | HSDAVFTDNYTRLRKQMAQKKYLNSIKNKR | 112 |
| rR3P110 | HSDAVFTDNYTRLRKQMARKKYLNSIKNKR | 113 |
| rR3P111 | HSDAVFTDNYTRLRKQMASKKYLNSIKNKR | 114 |
| rR3P112 | HSDAVFTDNYTRLRKQMATKKYLNSIKNKR | 115 |
| rR3P113 | HSDAVFTDNYTRLRKQMAVKKYLNSIKNKR | 116 |
| rR3P114 | HSDAVFTDNYTRLRKQMAWKKYLNSIKNKR | 117 |
| rR3P115 | HSDAVFTDNYTRLRKQMAYKKYLNSIKNKR | 118 |
| rR3P116 | HSDAVFTDNYTRLRKQMAAKKYLNSIANKR | 119 |
| rR3P117 | HSDAVFTDNYTRLRKQMAAKKYLNSICNKR | 120 |
| rR3P118 | HSDAVFTDNYTRLRKQMAAKKYLNSIDNKR | 121 |
| rR3P119 | HSDAVFTDNYTRLRKQMAAKKYLNSIENKR | 122 |
| rR3P120 | HSDAVFTDNYTRLRKQMAAKKYLNSIFNKR | 123 |
| rR3P121 | HSDAVFTDNYTRLRKQMAAKKYLNSIGNKR | 124 |
| rR3P122 | HSDAVFTDNYTRLRKQMAAKKYLNSIHNKR | 125 |
| rR3P123 | HSDAVFTDNYTRLRKQMAAKKYLNSIINKR | 126 |
| rR3P124 | HSDAVFTDNYTRLRKQMAAKKYLNSIMNKR | 127 |
| rR3P125 | HSDAVFTDNYTRLRKQMAAKKYLNSINNKR | 128 |
| rR3P126 | HSDAVFTDNYTRLRKQMAAKKYLNSIPNKR | 129 |
| rR3P127 | HSDAVFTDNYTRLRKQMAAKKYLNSIQNKR | 130 |
| rR3P128 | HSDAVFTDNYTRLRKQMAAKKYLNSIRNKR | 131 |
| rR3P129 | HSDAVFTDNYTRLRKQMAAKKYLNSISNKR | 132 |
| rR3P130 | HSDAVFTDNYTRLRKQMAAKKYLNSITNKR | 133 |
| rR3P131 | HSDAVFTDNYTRLRKQMAAKKYLNSIVNKR | 134 |
| rR3P132 | HSDAVFTDNYTRLRKQMAAKKYLNSIWNKR | 135 |
| rR3P133 | HSDAVFTDNYTRLRKQMAAKKYLNSIYNKR | 136 |
| rR3P134 | HSDAVFTDNYTRLRKQMAAKKYLNSIKNAR | 137 |
| rR3P135 | HSDAVFTDNYTRLRKQMAAKKYLNSIKNCR | 138 |
| rR3P136 | HSDAVFTDNYTRLRKQMAAKKYLNSIKNDR | 139 |
| rR3P137 | HSDAVFTDNYTRLRKQMAAKKYLNSIKNER | 140 |
| rR3P138 | HSDAVFTDNYTRLRKQMAAKKYLNSIKNFR | 141 |
| rR3P139 | HSDAVFTDNYTRLRKQMAAKKYLNSIKNGR | 142 |
| rR3P140 | HSDAVFTDNYTRLRKQMAAKKYLNSIKNHR | 143 |
| rR3P141 | HSDAVFTDNYTRLRKQMAAKKYLNSIKNIR | 144 |
| rR3P142 | HSDAVFTDNYTRLRKQMAAKKYLNSIKNLR | 145 |
| rR3P143 | HSDAVFTDNYTRLRKQMAAKKYLNSIKNMR | 146 |
| rR3P144 | HSDAVFTDNYTRLRKQMAAKKYLNSIKNNR | 147 |
| rR3P145 | HSDAVFTDNYTRLRKQMAAKKYLNSIKNPR | 148 |
| rR3P146 | HSDAVFTDNYTRLRKQMAAKKYLNSIKNQR | 149 |
| rR3P147 | HSDAVFTDNYTRLRKQMAAKKYLNSIKNRR | 150 |
| rR3P148 | HSDAVFTDNYTRLRKQMAAKKYLNSIKNSR | 151 |
| rR3P149 | HSDAVFTDNYTRLRKQMAAKKYLNSIKNTR | 152 |
| rR3P150 | HSDAVFTDNYTRLRKQMAAKKYLNSIKNVR | 153 |
| rR3P151 | HSDAVFTDNYTRLRKQMAAKKYLNSIKNWR | 154 |
| rR3P152 | HSDAVFTDNYTRLRKQMAAKKYLNSIKNYR | 155 |
| rR3P153 | HSDAVFTDNYTRLRKQMAAKKYLNSIKNKA | 156 |
| rR3P155 | HSDAVFTDNYTRLRKQMAAKKYLNSIKNKD | 157 |
| rR3P156 | HSDAVFTDNYTRLRKQMAAKKYLNSIKNKE | 158 |
| rR3P157 | HSDAVFTDNYTRLRKQMAAKKYLNSIKNKF | 159 |
| rR3P158 | HSDAVFTDNYTRLRKQMAAKKYLNSIKNKG | 160 |
| rR3P159 | HSDAVFTDNYTRLRKQMAAKKYLNSIKNKH | 161 |
| rR3P160 | HSDAVFTDNYTRLRKQMAAKKYLNSIKNKI | 162 |
| rR3P161 | HSDAVFTDNYTRLRKQMAAKKYLNSIKNKK | 163 |
| rR3P162 | HSDAVFTDNYTRLRKQMAAKKYLNSIKNKL | 164 |
| rR3P163 | HSDAVFTDNYTRLRKQMAAKKYLNSIKNKM | 165 |
| rR3P164 | HSDAVFTDNYTRLRKQMAAKKYLNSIKNKN | 166 |
| rR3P165 | HSDAVFTDNYTRLRKQMAAKKYLNSIKNKP | 167 |
| rR3P166 | HSDAVFTDNYTRLRKQMAAKKYLNSIKNKQ | 168 |
| rR3P167 | HSDAVFTDNYTRLRKQMAAKKYLNSIKNKS | 169 |
| rR3P168 | HSDAVFTDNYTRLRKQMAAKKYLNSIKNKT | 170 |
| rR3P169 | HSDAVFTDNYTRLRKQMAAKKYLNSIKNKV | 171 |
| rR3P170 | HSDAVFTDNYTRLRKQMAAKKYLNSIKNKW | 172 |
| rR3P171 | HSDAVFTDNYTRLRKQMAAKKYLNSIKNKY | 173 |
| R3P172 | HSDAVFTDNYTRLRKQVAAKKYLQSIKNKRYSWCEPGWCR | 174 |
| R3P173 | HSDAVFTDDYTRLRKEVAAKKYLESIKDKRY | 175 |
| PAC1 | ESDGIFTDSYSRYRKQMAVKKYLAAVL-NH₂ | 176 |
| PAC2 | HKDGIFTDSYSRYRKQMAVKKYLAAVL-NH₂ | 177 |
| PAC3 | HSKGIFTDSYSRYRKQMAVKKYLAAVL-NH₂ | 178 |
| PAC4 | HSDKIFTDSYSRYRKQMAVKKYLAAVL-NH₂ | 179 |
| PAC5 | HSDGKFTDSYSRYRKQMAVKKYLAAVL-NH₂ | 180 |
| PAC6 | HSDGIKTDSYSRYRKQMAVKKYLAAVL-NH₂ | 181 |
| PAC7 | HSDGIFKDSYSRYRKQMAVKKYLAAVL-NH₂ | 182 |
| PAC8 | HSDGIFTKSYSRYRKQMAVKKYLAAVL-NH₂ | 183 |
| PAC9 | HSDGIFTDKYSRYRKQMAVKKYLAAVL-NH₂ | 184 |
| PAC10 | HSDGIFTDSKSRYRKQMAVKKYLAAVL-NH₂ | 185 |
| PAC11 | HSDGIFTDSYKRYRKQMAVKKYLAAVL-NH₂ | 186 |
| PAC12 | HSDGIFTDSYSEYRKQMAVKKYLAAVL-NH₂ | 187 |
| PAC13 | HSDGIFTDSYSRKRKQMAVKKYLAAVL-NH₂ | 188 |
| PAC14 | HSDGIFTDSYSRYEKQMAVKKYLAAVL-NH₂ | 189 |
| PAC15 | HSDGIFTIDSYSRYREQMAVKKYLAAVL-NH₂ | 190 |
| PAC16 | HSDGIFTDSYSRYRKKMAVKKYLAAVL-NH₂ | 191 |
| PAC17 | HSDGIFTDSYSRYRKQKAVKKYLAAVL-NH₂ | 192 |
| PAC18 | HSDGIFTDSYSRYRKQMKVKKYLAAVL-NH₂ | 193 |
| PAC19 | HSDGIFTDSYSRYRKQMAKKKYLAAVL-NH₂ | 194 |
| PAC20 | HSDGIFTDSYSRYRKQMAVEKYLAAVL-NH₂ | 195 |
| PAC21 | HSDGIFTDSYSRYRKQMAVKEYLAAVL-NH₂ | 196 |
| PAC22 | HSDGIFTDSYSRYRKQMAVKKKLAAVL-NH₂ | 197 |
| PAC23 | HSDGIFTDSYSRYRKQMAVKKYKAAVL-NH₂ | 198 |
| PAC24 | HSDGIFTDSYSRYRKQMAVKKYLKAVL-NH₂ | 199 |
| PAC25 | HSDGIFTDSYSRYRKQMAVKKYLAKVL-NH₂ | 200 |
| PAC26 | HSDGIFTDSYSRYRKQMAVKKYLAAKL-NH₂ | 201 |
| PAC27 | HSDGIFTDSYSRYRKQMAVKKYLAAVK-NH₂ | 202 |
| rR3P174 | HSDAVFTDNYTRLRKQMAAKKYLNSIKNRI | 322 |
| rR3P175 | HSDAVFTDNYTRLRKQMAGKKYLNSIKNRI | 323 |
| rR3P176 | HSDAVFTDNYTRLRKQMAKKKYLNSIKNRI | 324 |
| rR3P177 | HSDAVFTDNYTRLRKQMARKKYLNSIKNRI | 325 |
| rR3P178 | HSDAVFTDNYTRLRKQMASKKYLNSIKNRI | 326 |
| rR3P179 | HSDAVFTDNYTRLRKQMAAKKYLNSIPNRI | 327 |
| rR3P180 | HSDAVFTDNYTRLRKQMAGKKYLNSIPNRI | 328 |
| rR3P181 | HSDAVFTDNYTRLRKQMAKKKYLNSIPNRI | 329 |
| rR3P182 | HSDAVFTDNYTRLRKQMARKKYLNSIPNRI | 330 |
| rR3P183 | HSDAVFTDNYTRLRKQMASKKYLNSIPNRI | 331 |
| rR3P184 | HSDAVFTDNYTRLRKQMAAKKYLNSIQNRI | 332 |
| rR3P185 | HSDAVFTDNYTRLRKQMAGKKYLNSIQNRI | 333 |
| rR3P188 | HSDAVFTDNYTRLRKQMAKKKYLNSIQNRI | 334 |
| rR3P187 | HSDAVFTDNYTRLRKQMARKKYLNSIQNRI | 335 |
| rR3P188 | HSDAVFTDNYTRLRKQMASKKYLNSIQNRI | 336 |
| rR3P189 | HSDAVFTDNYTRLRKQMAAKKYLNSIRNRI | 337 |
| rR3P190 | HSDAVFTDNYTRLRKQMAGKKYLNSIRNRI | 338 |
| rR3P191 | HSDAVFTDNYTRLRKQMAKKKYLNSIRNRI | 339 |
| rR3P192 | HSDAVFTDNYTRLRKQMARKKYLNSIRNRI | 340 |
| rR3P193 | HSDAVFTDNYTRLRKQMASKKYLNSIRNRI | 341 |

### Example 8. Insulin secretion by rat islets

Peptide R3P3 (0.1-100 nM) stimulates insulin secretion from isolated rat islets in a glucose-dependent fashion. These studies compare the effects on islet cells of R3P3 peptide and GLP-1.

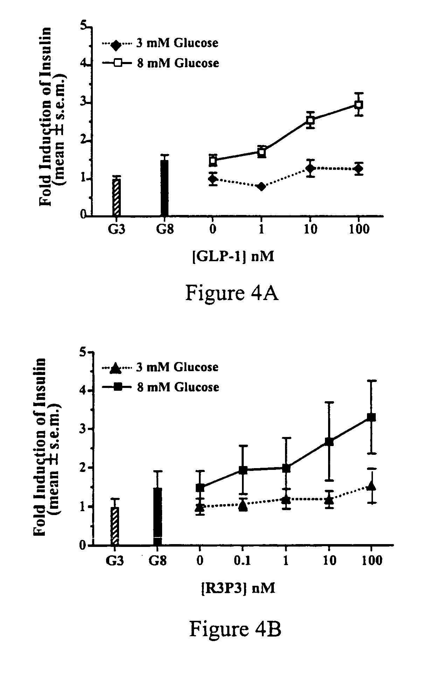

Rat islets were isolated and treated with GLP-1 or R3P3 at either 3 or 8 mM glucose in the medium in accordance with the rat islet protocol described above in example 1. As shown by Figures 4A-4B, peptide R3P3 which is not part of the present invention significantly enhances the release of insulin from islets in a concentration-dependent fashion and this effect is similar to that of GLP-1, a known insulin secretagogue.

### Example 9. In vivo insulin and glucose response

As shown in Tables 2 and 3 below, polypeptides that activate the R3 receptor also potentiate glucose-induced increase in plasma insulin levels when compared to glucose alone. This increase in insulin causes concomitant decrease in plasma glucose.

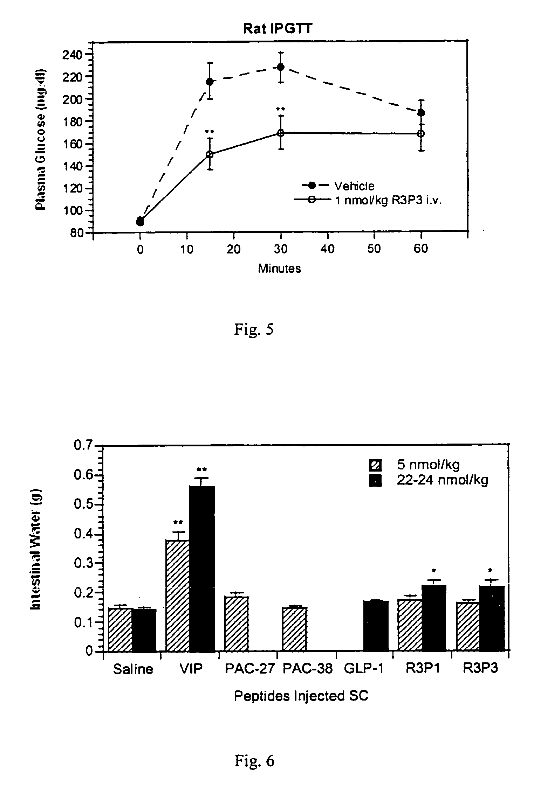

In accordance with the protocol of example 2 above, overnight fasted Wistar rats were anesthetized with pentobarital; i.v. injected with glucose ± peptide and eye bled after 1 minute. N=12 rats/group. The graph in Figure 5 shows that the polypeptide R3P3 which is not provided by the present invention causes an increase in glucose disposal that accompanies the increase in insulin secretion. The peptide or vehicle was given i.v. followed by a glucose load given i.p. in accordance with example 3. The plasma glucose was followed over the time period indicated. As shown in the graph, R3P3 markedly accelerated the disposal of blood glucose.

### Example 10. Diarrhea side effects.

As described in the protocol of example 4 above, fasted rats were injected s.c. with the indicated peptide (5 nmol/kg or 22-24 nmol/kg). Five minutes after the injection, 0.3 ml of water was given p.o. Five min after the water dose, the animals were euthanized and the water content In the small intestine determined. As shown in Figure 6, VIP injections at the two doses caused a marked increase in the water content of the small intestine lumen over the vehicLe (saline) control. At the highest dose, the R3 peptides caused only a very small increase (i.e., roughly 10%) in comparison to VIP. At the 5 nmol/kg dose, the peptides did not produce any change in water content in the small gut. The degree of water retention was used as an index of R2 activation *in vivo*.

### Example 11. Effect of Peptides on Intraperitoneal Glucose Tolerance in Rats

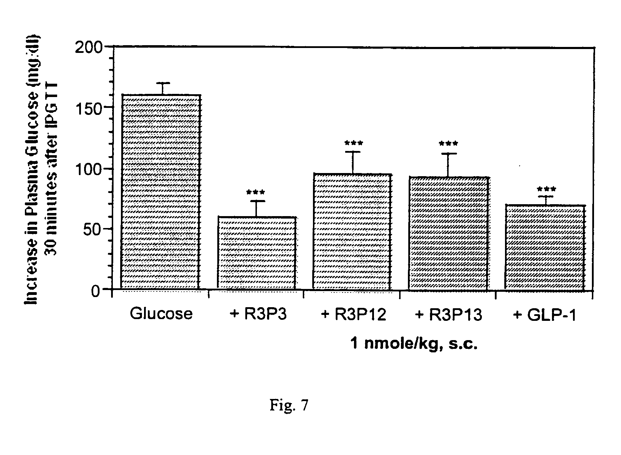

Wistar rats were fasted overnight and then anesthetized with Pentobarbital. The rats were eye-bled (zero time) and the peptide (in 1 % human albumin) was injected subcutaneously. Five minutes later, 1 g/kg of glucose (in saline) was injected intraperitoneally, and the rats were eye-bled after 30 minutes. Plasma glucose levels were determined using the Axon autoanalyzer, and are shown in Figure 7.

Figure 7 shows that plasma glucose elevated to 160 mg/dl above basal (100mg/dl) in rats treated with vehicle 30 minutes after IPGTT (IP Glucose Tolerance Test). In rats injected with peptides R3P3, R3P12 and R3P13 which are not part of the present invention, this elevation of plasma glucose was significantly reduced, verifying the Insulin-producing effect. At 1 nmole/kg s.c. the glucose-lowering effect of each peptide was similar to that observed with an equivalent dose of GLP-1.

### Example 12. Glucose-dependent insulin secreting polypeptides.

Table 2 contains a list of the peptides that stimulated release of insulin in vivo in the IPGTT assay, or in vitro through the rat islet assay. As shown by the data, the peptides enhance glucose-mediated insulin release in vivo and in vitro.

Plasma insulin: Data are expressed as % of plasma insulin one minute after IVGTT (0.4g/kg glucose) with either saline, 0.1nmol/kg P51, P55, P60, P66 or 1 nmole/kg of the other peptides including peptides which are not part of the present invention in Wistar rats. Blood was drawn from the eye and insulin was measured with the rat insulin RIA kit (Linco Research, Inc, St. Charles, MO).

Plasma glucose: Data are expressed as % of vehicle of plasma glucose area under the curve after IPGTT (1 g/kg glucose) after treatment with 1 nmol/kg dose of the peptide. PACAP27 has been reported to induce insulin secretion but does not affect plasma glucose levels (Filipsson, K. et al., J. Clin. Endocrin. & Metabolism 82: 3093-3098 (1997)). The present Inventors have shown for the first time that this is because the effects of R2 and R3 on glucose tend to cancel each other out. The R2-selective agonist [K15, R16, L27]VIP(1-7)/GRF(8-27) (Gourlet, P. at al., Peptides 18:1539-45 (1997)), defined as R2P1, increases plasma glucose level by 14% while R3-selective agonists lower plasma glucose level by -20%. Therefore, R3-selectlvlty appears to be a desirable attribute for medicaments which are to be employed in achieving blood glucose reduction, for treatment of type 2 diabetes.

Islet insulin release: Rat islets were isolated from Sprague-Dawley rats as described in example 1 and treated with either vehicle or specified peptide (10nM) for 2hrs. Insulin concentration in the medium was measured with the Linco rat insulin RIA kit. Glucose concentration in the medium was 8mM. The data are expressed as % of [insulin] in 8mM glucose alone. No polypeptide-induced Increases in insulin concentration were observed al 3 mM glucose; thus, the insulin releasing activity of these polypeptides are glucose-dependent. It should be noted that the following Table includes data for polypeptides which are not part of the present invention.

**Table 2**

| Peptide | Plasma Insulin (% of Basal) | Plasma Glucose (% of Basal) | | Islet Insulin Release (% of Basal) |
|---|---|---|---|---|
| | | i.v. | s.c. | |
| PACAP27 | 320 | | | 186 |
| R2P1 | 216 | 114 | | |
| R3P0 | | 77 | | 264 |
| R3P1 | 480 | 73 | | 250 |
| R3P3 | 361 | 77 | 72 | 275 |
| R3P9 | | | | 221 |
| R3P10 | | | | 174 |
| R3P12 | 302 | | 76 | 324 |
| R3P13 | 465 | | 77 | 170 |
| R3P19 | 285 | | | |
| R3P36 | 255 | 73 | 78 | |
| P51 | 259 | | | |
| P55 | 208 | | | |
| P60 | 283 | | | |
| R3P66 | 388 | | 82 | 184 |
| R3P77 | 388 | | | |
| R3P80 | 360 | | | |
| R3P81 | 302 | | | |

### Example 13. Pharmaceutical composition - IV formulation

A sterile injectable formulation is made from 4 mg of a polypeptide of SEQ ID NO: 72 and 1 liter of sterile saline, using a manufacturing process well known in the art.

### Example 14. Pharmaceutical composition - IV formulation

A sterile injectable formulation which is not part of the present invention is made from 400 mg of a polypeptide of SEQ ID NO 174 and 1 liter of sterile saline, using a manufacturing process well known In the art. Example 15: Effect of PACAP27, VIP and PACAP receptor-selective peptide agonists on heart rate in conscious dogs

### Protocol:

Beagle dogs were put into a sling where they have been trained to stand for up to 3 hours. The cuff of the heart rate monitor was placed around the tail of the dog.

Saline was injected into the cephalic vein and heart rate monitored every 2 minutes to establish a baseline. After 10 minutes, peptide, including peptides which are not part of the invention, was injected and heart rate monitored every 2 minutes for the next 20 minutes. If heart rate was normal at that time, a higher dose was given and heart rate monitored for 20 minutes. The area under the curve (AUC) for the first 10 minutes of heart rate change induced by the polypeptide was plotted as % over vehicle AUC against polypeptide concentration. PACAP27 and the R1-selective agonist maxadilan (Moro, O. J. Biol. Chem. 272:966-970 (1997)) possess similar potency in heart rate increase. VIP which activates both R2 and R3 but not R1, R2-selective agonist R2P1, and R3 selective agonists R3P0, R3P3, R3P19, R3P36, R3P51 and R3P53 are at least 10-fold less potent than PACAP27 or mazadilan. Thus, the cardiovascular effect of PACAP27 can be mostly attributed to PACAP-R1 activation. All peptides are full agonists at their respective receptors with comparable affinities. R3P0 is the Roche analog RO 25-1553 that has been shown to display selectively for PACAP-R3 of at least 100-fold over R1 and R2 (Gourlet et al, Peptides 18:4030408 (1997)).

### Example 16. Cyclic AMP SPA

CHO cells expressing the PACAP R3 were plated in 96-well plates (Costar) at 8 x 10⁴ cell/well and grown at 37C for 24 hours in a MEM + nucleosides + glutamine (Giobco BRL), 10% FBS, 100 µg/ml Pen/Strep, 0.3 mg/ml glutamine, 1 mM HEPES, 0.5 mg/ml Geneticin (Gibco BRL). The media was removed and the plates were washed with PBS. The cells were incubated with a peptide, in Hepes-PBS-BSA with 0.4mg/mlSoybean Trypsin inhibitor, 0.5 mg/ml Bacitracin, 100uM IBMX, for 15 min at 37 C. Cyclic AMP in the ceii extracts was quantitated using the cAMP SPA direct screening assay system (Amersham Pharmacia Biotech Inc, Piscataway, NJ,). The peptides shown in Table 3 were assayed for cAMP activity.

Table 3 reports *in vitro* cAMP SPA results on CHO cells transfected with PACAP-R2 or PACAP-R3. EC50 is defined as the concentration of the polypeptide at which 50% of maximum PACAP27 activity is achieved. "NA" denotes no detectable activities. "R3 Selectivity" is derived from the ratio of EC50 at R2 versus EC50 at R3. Most of the following polypeptides are designed based on VIP, which has been shown to lack activity at R1 (Vaudry D. et al., 2000, Pharmacological Reviews, 52: 269-324). Therefore, it is believed that these polypeptides do not possess appreciable activity at R1. Peptides, and sequences which are not part of the invention, designed based on VIP include SEQ ID NO: 6-53, 62-65 and 70-175. Table 3 includes some peptides which are not part of the present invention.

**Table 3**

| Peptide | Seq ID NO | R2 EC50, nM | R3 EC50, nM | R3 Selectivity |
|---|---|---|---|---|
| VIP | 1 | 0.1 | 0.08 | 1.3 |
| R3P0 | 5 | >100 | 0.4 | >250 |
| R3P1 | 6 | >100 | 2 | >50 |
| R3P2 | 7 | >1000 | 3 | >300 |
| R3P3 | 8 | 100 | 0.75 | 130 |
| R3P5 | 10 | 200 | 7 | 30 |
| R3P8 | 11 | 2 | 1.4 | 1.5 |
| R3P9 | 12 | 40 | 2 | 20 |
| R3P10 | 13 | 3 | 11 | 0.3 |
| R3P11 | 14 | 10 | 3 | 3 |
| R3P12 | 15 | >100 | 0.5 | >200 |
| R3P13 | 16 | 163 | 5 | 33 |
| R3P14 | 17 | 50 | 17 | 3 |
| R3P19 | 18 | 42 | 1.4 | 30 |
| R3P20 | 19 | 330 | 1.4 | 230 |
| R3P21 | 20 | 17 | 0.3 | 60 |
| R3P22 | 21 | 38 | 1.6 | 24 |
| R3P24 | 22 | 45 | 1.3 | 34 |
| R3P25 | 23 | 15 | 0.7 | 20 |
| R3P26 | 24 | >100 | 0.55 | >180 |
| R3P29 | 25 | >100 | 0.5 | >200 |
| R3P30 | 26 | 20 | 0.4 | 50 |
| R3P31 | 27 | >100 | 0.3 | >300 |
| R3P32 | 28 | 84 | 0.4 | 200 |
| R3P33 | 29 | >100 | 0.5 | >200 |
| R3P34 | 30 | >100 | 1.4 | >70 |
| R3P35 | 31 | >100 | 2.6 | >40 |
| R3P36 | 32 | >90 | 0.4 | >200 |
| R3P41 | 33 | 8 | 0.08 | 100 |
| R3P42 | 34 | 0.3 | 0.03 | 10 |
| R3P43 | 35 | 0.8 | 0.08 | 10 |
| R3P44 | 36 | 8 | 1.3 | 6 |
| R3P45 | 37 | NA | NA | |
| R3P46 | 38 | 0.4 | 0.06 | 7 |
| R3P47 | 39 | 0.7 | 0.12 | 6 |
| R3P48 | 40 | 1 | 0.13 | 8 |
| R3P49 | 41 | 8 | 0.27 | 30 |
| R3P50 | 42 | 0.7 | 0.13 | 6 |
| R3P51 | 43 | 23 | 0.26 | 90 |
| R3P52 | 44 | >100 | 0.4 | >250 |
| R3P53 | 45 | 500 | 0.2 | 2500 |
| R3P54 | 46 | >100 | 0.5 | >200 |
| R3P55 | 47 | 50 | 0.17 | 280 |
| R3P56 | 48 | 40 | 0.4 | 100 |
| R3P57 | 49 | 42 | 0.23 | 190 |
| R3P58 | 50 | 55 | 0.32 | 170 |
| R3P59 | 51 | 10 | 0.15 | 70 |
| R3P60 | 52 | 120 | 1 | 120 |
| R3P61 | 53 | 110 | 0.8 | 140 |
| R3P6 | 57 | 220 | 80 | 3 |
| R3P7 | 58 | 2 | 4 | 0.5 |
| R3P15 | 59 | 12 | 5 | 2 |
| R3P16 | 60 | 14 | 10 | 1.4 |
| R3P17 | 61 | 9 | 4 | 2 |
| R3P18 | 62 | NA | NA | |
| R3P23 | 63 | 10 | 0.43 | 23 |
| R3P27 | 64 | 6.4 | 0.8 | 8 |
| R3P28 | 65 | 8 | 7 | 1 |
| R3P37 | 66 | 71 | 4 | 18 |
| R3P38 | 67 | 17 | 2.3 | 7 |
| R3P39 | 68 | 1 | 0.5 | 2 |
| R3P40 | 69 | 1.4 | 0.7 | 2 |
| R3P62 | 70 | 180 | 1.8 | 100 |
| R3P65 | 71 | 80 | 0.6 | 130 |
| R3P66 | 72 | 100 | 0.4 | 250 |
| R3P67 | 73 | 100 | 0.3 | 330 |
| R3P68 | 74 | 130 | 0.5 | 260 |
| R3P69 | 75 | 90 | 0.5 | 190 |
| R3P70 | 76 | >100 | 0.4 | >250 |
| R3P71 | 77 | 56 | 0.09 | 660 |
| R3P72 | 78 | >100 | 0.16 | >600 |
| R3P73 | 79 | 50 | 0.3 | 170 |
| R3P74 | 80 | 90 | 0.6 | 150 |
| R3P75 | 81 | >150 | 0.3 | >500 |
| R3P76 | 82 | >150 | 0.1 | >1500 |
| R3P77 | 83 | 200 | 0.4 | 500 |
| R3P78 | 84 | 250 | 1.1 | 230 |
| R3P79 | 85 | 100 | 0.5 | 200 |
| R3P80 | 86 | 88 | 0.44 | 200 |
| R3P81 | 87 | 50 | 0.5 | 100 |
| R3P82 | 88 | 10 | 0.4 | 23 |
| R3P83 | 89 | 5 | 0.08 | 60 |
| R3P84 | 90 | 2.5 | 0.06 | 40 |
| R3P85 | 91 | 5 | 0.18 | 30 |
| R3P86 | 92 | 90 | 0.8 | 110 |
| R3P87 | 93 | 0.6 | 0.2 | 3 |
| R3P88 | 94 | 0.9 | 0.08 | 12 |
| R3P89 | 95 | 0.9 | 0.06 | 15 |
| R3P92 | 98 | 2 | 0.02 | 100 |
| R3P93 | 98 | 6 | 0.14 | 40 |
| R3P94 | 99 | 9 | 0.09 | 100 |
| R3P98 | 101 | NA | NA | |
| R3P99 | 102 | 40 | 1.0 | 40 |
| R3P100 | 103 | 10 | 0.3 | 30 |
| R3P101 | 104 | 400 | 1.0 | 400 |
| R3P102 | 105 | 300 | 60.0 | 5 |
| R3P103 | 106 | 0.4 | 0.05 | 8 |
| R3P104 | 107 | 100 | 0.2 | 500 |
| R3P105 | 108 | 1 | 0.3 | 3 |
| R3P106 | 109 | 2 | 0.1 | 20 |
| R3P107 | 110 | 1000 | 200.0 | 5 |
| R3P108 | 111 | 1000 | 300.0 | 3 |
| R3P109 | 112 | 1000 | 10.0 | 100 |
| R3P110 | 113 | 100 | 0.5 | 200 |
| R3P111 | 114 | 1000 | 3.0 | 300 |
| R3P112 | 115 | 4 | 0.1 | 40 |
| R3P113 | 116 | 3 | 0.3 | 10 |
| R3P114 | 117 | 20 | 2.0 | 10 |
| R3P115 | 118 | 300 | 6.0 | 50 |
| R3P116 | 119 | 20 | 1.0 | 20 |
| R3P118 | 121 | 15 | 0.5 | 30 |
| R3P119 | 122 | 15 | 0.5 | 30 |
| R3P120 | 123 | 10 | 0.2 | 50 |
| R3P121 | 124 | 50 | 1.0 | 50 |
| R3P122 | 125 | 10 | 0.2 | 50 |
| R3P123 | 126 | 5 | 0.1 | 50 |
| R3P124 | 127 | 3 | 0.2 | 15 |
| R3P125 | 128 | 60 | 2.0 | 30 |
| R3P126 | 129 | 200 | 1.0 | 200 |
| R3P127 | 130 | 300 | 0.5 | 600 |
| R3P128 | 131 | 60 | 0.3 | 200 |
| R3P129 | 132 | 50 | 1.0 | 50 |
| R3P130 | 133 | 40 | 1.0 | 40 |
| R3P131 | 134 | 20 | 0.3 | 70 |
| R3P132 | 135 | 10 | 0.2 | 50 |
| R3P133 | 136 | 8 | 0.1 | 80 |
| R3P134 | 137 | 40 | 0.4 | 100 |
| R3P 136 | 139 | 20 | 0.3 | 70 |
| R3P137 | 140 | 30 | 0.3 | 100 |
| R3P139 | 142 | 20 | 0.4 | 50 |
| R3P140 | 143 | 15 | 0.3 | 50 |
| R3P141 | 144 | 20 | 0.2 | 100 |
| R3P142 | 145 | 6 | 0.2 | 30 |
| R3P143 | 146 | 6 | 0.2 | 30 |
| R3P144 | 147 | 300 | 3.0 | 100 |
| R3P145 | 148 | 50 | 0.5 | 100 |
| R3P146 | 149 | 30 | 0.3 | 100 |
| R3P147 | 150 | 100 | 0.2 | 500 |
| R3P148 | 151 | 30 | 0.3 | 100 |
| R3P149 | 152 | 40 | 0.5 | 80 |
| R3P150 | 153 | 40 | 0.8 | 50 |
| R3P153 | 156 | 40 | 0.2 | 200 |
| R3P155 | 157 | 40 | 0.4 | 100 |
| R3P156 | 158 | 100 | 1.0 | 100 |
| R3P157 | 159 | 50 | 0.3 | 170 |
| R3P158 | 160 | 6 | 0.07 | 90 |
| R3P159 | 161 | 200 | 0.5 | 400 |
| R3P160 | 162 | 100 | 0.2 | 500 |
| R3P161 | 163 | 60 | 0.2 | 300 |
| R3P162 | 164 | 20 | 0.1 | 200 |
| R3P163 | 165 | 40 | 0.2 | 200 |
| R3P164 | 166 | 100 | 0.3 | 300 |
| R3P165 | 167 | 150 | 0.5 | 300 |
| R3P166 | 168 | 50 | 0.1 | 500 |
| R3P167 | 169 | 300 | 1.0 | 300 |
| R3P168 | 170 | 60 | 0.2 | 300 |
| R3P169 | 171 | 60 | 0.2 | 300 |
| R3P170 | 172 | 20 | 0.1 | 200 |
| R3P171 | 173 | 80 | 0.4 | 200 |
| R3P172 | 174 | 77 | 2.6 | 29 |
| R3P173 | 175 | NA | 200 | |
| PAC1 | 176 | 970 | 10 | 97 |
| PAC2 | 177 | NA | 34 | |
| PAC3 | 178 | NA | NA | |
| PAC4 | 179 | 45 | 7 | 6 |
| PAC5 | 180 | 1.8 | 0.7 | 2 |
| PAC6 | 181 | NA | NA | |
| PAC7 | 182 | NA | NA | |
| PAC8 | 183 | 43 | 47 | 1 |
| PAC9 | 184 | 0.9 | 0.7 | 1 |
| PAC10 | 185 | 110 | 27 | 4 |
| PAC11 | 186 | 10 | 140 | 0.1 |
| PAC12 | 187 | 150 | 3 | 50 |
| PAC13 | 188 | 3 | 0.5 | 6 |
| PAC14 | 189 | 110 | 1.6 | 70 |
| PAC15 | 190 | 2.4 | 0.2 | 12 |
| PAC16 | 191 | 0.2 | 0.2 | 1 |
| PAC17 | 192 | 0.25 | 0.15 | 1.6 |
| PAC18 | 193 | 0.7 | 1.1 | 0.7 |
| PAC19 | 194 | 8 | 0.4 | 20 |
| PAC20 | 195 | 20 | 0.7 | 30 |
| PAC21 | 196 | 2.5 | 0.24 | 10 |
| PAC22 | 197 | 2 | 15 | 0.1 |
| PAC23 | 198 | 170 | 13 | 13 |
| PAC24 | 199 | 0.3 | 0.2 | 1.5 |
| PAC25 | 200 | 0.13 | 0.04 | 3 |
| PAC26 | 201 | 0.25 | 0.3 | 1 |
| PAC27 | 202 | 1.5 | 1.1 | 1.4 |
| rR3P174 | 322 | 18 | 0.20 | 90 |
| rR3P175 | 323 | 400 | 2.2 | 180 |
| rR3P176 | 324 | 300 | 2.0 | 150 |
| rR3P177 | 325 | 110 | 1.6 | 70 |
| rR3P178 | 326 | 80 | 0.75 | 110 |
| rR3P179 | 327 | 230 | 1.5 | 150 |
| rR3P180 | 328 | >100 | 6.7 | >20 |
| rR3P181 | 329 | 280 | 5.1 | 50 |
| rR3P182 | 330 | 280 | 3.2 | 90 |
| rR3P183 | 331 | >150 | 5.4 | >30 |
| rR3P184 | 332 | 10 | 0.37 | 30 |
| rR3P185 | 333 | 180 | 4.5 | 40 |
| rR3P186 | 334 | 70 | 1.6 | 44 |
| rR3P187 | 335 | >130 | 1.6 | >80 |
| rR3P188 | 336 | 150 | 2.2 | 70 |
| rR3P189 | 337 | 1.3 | 0.04 | 30 |
| rR3P190 | 338 | 220 | 2.2 | 100 |
| rR3P191 | 339 | >200 | 2.7 | >80 |
| rR3P192 | 340 | 40 | 0.60 | 60 |
| rR3P193 | 341 | 200 | 1.9 | 110 |

### Example 17. Polyclonal antibody production

Synthesis of the peptide Ac-CRKQVAAKKYLQSIKNKRY COOH was performed on an Applied Biosystems 430A peptide synthesizer using fmoc chemistry with HBTU activation of amino acids. The peptide was cleaved using a 84.6% TFA, 4.4% phenol, 4.4% water, 4.4% thioanusol, and 2.2% ethandithiol cocktail. The crude peptide was purified using a C18 reverse phase column with a 0.1%TFA/CH3CN gradient. Evaluation of purity was performed on a PerSeptive V Biosystems Voyager DE Pro MALDI mass spectrometer. The cysteine residue was coupled to KLH using the Pierce Imject Maleimide Activated mcKLH kit and protocol (Pierce, Rockford, IL). Rabbits were immunized using the following polyclonal antiserum immunization schedule:
Day 0 - 10 ml prebleed for baseline serum
   250ug each peptide in 1 ml emulsion of Complete Freunds Adjuvant,
   0.1 ml/site X 10 sites subcutaneously
Day 14 - Boost 250 µg each peptide in 1 ml emulsion of incomplete Freunds Adjuvant
   0.1 ml/site X 10 sites subcutaneously
Day 21 - 35 ml bleed
Day 35 - Boost 250 µg each peptide in 1 ml emulsion of Incomplete Freunds Adjuvant
   0.1 ml/site X 10 sites subcutaneously
Day 42 - 35 ml bleed
Day 56 - Boost 250 µg each peptide in 1 ml emulsion of Incomplete Freunds Adjuvant
   0.1 ml/site X 10 sites subcutaneously
Day 63 - 35 ml bleed
Day 77 - Boost 250 µg each peptide in 1 ml emulsion of Incomplete Freunds Adjuvant
   0.1 ml/site X 10 sites subcutaneously
Day 84 - Terminal bleed

### Antibodies were characterized using the following protocol:

Immulon III plate (DYNATECH LABORATORIES, INC, Chantilly, Virginia) was coated with P66 peptide (0.3-100ng range) in 100ul of EIA coating buffer 1.6L of 0.1 M NaHCO3 + 0.4L of 0.1 M Na2CO3 pH9.5) for 3 hours at room temperature (RT). The plate was blotted with 100ul of 5% milk TBS Tween 20 (10mM tris pH8.0, 150mM NaCl, 0.05% Tween-20 (SIGMA P-1379)) for 1 hour at RT and washed 3 times with TBS Tween. R3P66 antibody was added to the well in 100ul of 5% bloto (TBS- Tween 20 + 5% milk ) for 2 hours RT followed by a wash with TBS Tween (repeat wash 5 times). Secondary antibody (BioRad goat anti rabbit Alkaline Phosphatase conjugate) was added to the well at 1:1000 dilution in 100ul of 5% bloto for 1 hour. The plate was washed 5 times with TBS Tween. p-Nitrophenyl Phosphate (SIGMA 104-105) 0.5mg/ml of substrate buffer (1 M Diethanolamine, 0.5uM MgCl2*6H2O, pH 9.8 w/HCl ) in 100ul and incubated at RT for1 hour to O/N. The plate was read at OD 405 in SPECTRAmax 250 (Molecular Devices Corporation, Sunnyvale, CA).

### To determine if the antibodies produced in rabbits to the peptide

Ac-CRKQVAAKKYLQSIKNKRY-COOH in accordance with example 17 recognize the peptide R3P66 (SEQ ID NO 72), the enzyme-linked immunoadsorbent assay (ELISA) was performed. When antibodies recognize a peptide, a signal at OD405 is detected. Figure 10 shows that these antibodies recognize R3P66 but do not interact with homologous peptides PACAP-27 or VIP up to 30ug of peptide concentration.

### Example 18: Airway Hyperresponsiveness in the Primate Acute Asthma Model

Male cynomolgus monkeys (*Macaca fascicularis)* used in this study were maintained at constant temperature and humidity, with a twelve hour light cycle. They were fed twice daily, except on an experimental day when food was withheld the night before the procedure. Water was available *ad lib* at all times.

Airway hyperresponsiveness (AHR)(baseline) was measured 1 week before the control (no treatment) antigen challenge, and again 24hrs after antigen challenge. Antigen-induced airway hyperresponsiveness was measured by a fall in the PC₁₀₀ (the concentration of methacholine required to cause a 100% increase in lung resistance) at 24hr compared to baseline. After 2 weeks rest, another baseline measurement of AHR was performed. One week later, peptides of this invention were administered as an aerosol, 10 minutes before antigen challenge. After 24hrs. AHR was again measured, and the fall in PC₁₀₀ with treatment was compared to that without treatment.

Experimental Procedure. On each experimental day animals were anaesthetized with a ketamine/xylazine mixture (70:12 mg kg⁻¹ @ 0.1 ml kg⁻¹) while still in their cage. When unconscious they were brought into the primate laboratory where they were placed in a supine position on a heated water blanket on a trolley. Ophthalmic ointment was wiped onto each eye, and 0.2ml lidocaine (2%,) sprayed onto the larynx and over the back of the throat. The jaws were held apart by a jaw spreader and a cuffed 5.0 gauge endotracheal tube (with the end liberally smeared with xylocaine gel, 2%) was inserted with the aid of laryngoscope. The animal was then placed into a specially designed restraint chair such that the animal was in a slightly reclined but upright sitting position, secured only by a collar at the neck. A water-heated blanket surrounded the animal.

The endotracheal tube was connected to a Harvard Ventilator adjusted to deliver 30-35 breaths per minute. Airflow was measured by a Fleisch pneumotachograph and thoracic pressure was measured by a validyne pressure transducer (as the difference between the pressure at the distal end of the et tube and room pressure).

The pneumotachograph and validyne were connected to a pre-amplifier and then into an MI² respiratory analyser. Using the primary signals of flow and pressure the analyser computed airway resistance and compliance (as well as a number of other respiratory parameters). An initial measurement of 5-6 minutes was carried out to ensure the signals were steady and that the values for resistance and compliance were within recognized limits.

Antigen challenge: This was an inhalation challenge with *Ascaris suum.* The supplied *Ascaris suum* extract was diluted tenfold with PBS to give a 1 000ug/ml solution. The aerosol was delivered with a pressure driven Rainbow drop nebuliser (Puritan-Bennett) connected to a Bird mark 7A respirator, set to deliver 15 breaths per minute. 30 breaths of antigen were administered after which the acute bronchoconstriction was monitored for 15min.

After the challenge had been finished the animal was weaned off the ventilator, and when he could breath for himself was released from the restraint chair and laid supine on the trolley, When the normal reflexes (eye blink, swallow) had returned, along with muscle tone in the limbs the animal was returned to its cage.
Peptide Administration: Peptides under evaluation were delivered by inhalation as above. The following list of peptides includes peptides which are not part of the present invention. Stock solutions of the peptides were diluted with sterile water to achieve a concentration of 0.5ug/4ul. From previous measurements the nebuliser had been demonstrated to deliver 4ul/breath, and the number of breaths administered to each animal was adjusted to deliver the correct final concentration from the nebuliser. For each peptide the final concentrations delivered were as follows:
R3P0 (SEQ ID NO:5) -0.6ug/kg
R3P76 (SEQ ID NO:82) - 3ug/kg
R3PB2 (SEQ **I**D NO:88) - 1.8ug/kg

Methacholine challenge: Methacholine dose response curves were carried out to assess the airway hyperresponsiveness. In the acute model, this was measured at +24 hour and compared to the responsiveness 7 days before treatment. An aerosol of phosphate buffered saline (PBS) was delivered using a nebuliser as above. The aerosol was administered for 15 breaths and then lung resistance was monitored for ten minutes. Methacholine (Sigma) was made up at a concentration of 100mg/ml in PBS and this stock solution was diluted with PBS to a final range of concentrations from 0.1mg/ml through to 100mg/ml. Methacholine (0.1mg/ml, 15 breaths) was administered followed by another ten minutes monitoring. Successive doses of methacholine were administered with the dose increasing by a half-log at each step until either the lung resistance had doubled or the maximum dose of methacholine (100mg/ml)had been administered. The baseline (zero %) resistance was taken as the resistance achieved following the PBS administration. The increase in lung resistance (%) and the methacholine doses were entered into a spreadsheet and the PC₁₀₀ (the dose of methacholine to cause a 100% increase in resistance) was calculated from a graph of dose against resistance. These values were converted to log₁₀ values. The delta PC₁₀₀ (+24hr value - baseline value) for the treated study was compared to that for the control study.

Table 4 shows the PC₁₀₀ data for the three peptides. As shown by the data, the peptides are effective against antigen-induced airway hyperresponsiveness, and are thus likely to be a potential asthma therapy. It should be noted that R3P0 (SEQ ID NO: 5) and R3P82 (SEQ ID NO: 88) are not part of the present invention.

**Table 4: PC₁₀₀ data for all three peptide studies.**

| | Peptide (dose) | | | | | |
|---|---|---|---|---|---|---|
| | R3P0 (0.6ug/mg) | | R3P76 (3.0ug/kg) | | R3P82 (1.8ug/kg) | |
| | Control | Treated | Control | Treated | Control | Treated |
| Delta log₁₀ PC₁₀₀ | | | | | | |
| N | 4 | 4 | 5 | 5 | 4 | 4 |
| Mean* | -0.325 | -0.150 | -0.468 | +0.047 | -0.567 | -0.067 |
| SD | 0.177 | 0.152 | 0.128 | 0.253 | 0.361 | 0.329 |
| P(Treated vs Control) | 0.240 | | 0.031 | | 0.087 | |
| % Inhibition of induced AHR | 54 | | 100 | | 88 | |

| | | | | | | |
|---|---|---|---|---|---|---|
| *The larger the negative value, the more hyperresponsive the animal becomes. | | | | | | |

All publications and patents mentioned in the above specification are incorporated herein by reference. Various modifications and variations of the described compositions and methods of the invention will be apparent to those skilled in the art without departing from the scope and spirit of the invention. Although the invention has been described in connection with specific preferred embodiments, It should be understood that the invention as claimed should not be unduly limited to such specific embodiments. Indeed, various modifications of the above-described modes for carrying out the invention which are obvious to those skilled in the field of molecular biology or related fields are intended to be within the scope of the following claims. Those skilled in the art will recognize, or be able to ascertain using no more than routine experimentation, many equivalents to the specific embodiments of the invention described herein. Such equivalents are intended to be encompassed by the following claims.

## Claims

1. A polypeptide selected from the group consisting of SEQ ID NOs: 47, 72-82 and 85.

2. A polynucleotide encoding a polypeptide sequence of claim 1, or a degenerate variant thereof.

3. A vector comprising a polynucleotide of claim 2.

4. An isolated host cell comprising a vector of claim 3.

5. A method for producing a polypeptide of claim 1 comprising:
a) culturing the host cell of claim 4 under conditions suitable for the expression of said polypeptide; and
b) recovering the polypeptide from the host cell culture.

6. A pharmaceutical composition comprising a polypeptide of claim 1 in combination with a pharmaceutically acceptable carrier.

7. A gene therapy composition comprising a polynucleotide of claim 2 in combination with a therapeutically effective gene therapy vector.

8. The polypeptide of claim 1, wherein said polypeptide is represented by SEQ ID NO: 72.

9. A purified antibody which binds specifically to the polypeptide of claim 1.

10. Use of a PACAP R3 agonist selected from the group consisting of SEQ ID NOs: 47, 72-82 and 85 for the manufacture of a medicament for treating a metabolic disorder in a mammal.

11. Use according to claim 10, wherein the PACAP R3 agonist has at least about 100-fold selectively for PACAP R3 over PACAP R2 or PACAP R1.

12. Use according to any of claims 10 to 11, wherein the R3 agonist is the polypeptide of SEQ ID NO: 72.

13. Use according to any of claims 10 to 12, wherein said metabolic disorder is type 2 diabetes.

14. Use according to any of claims 10 to 13, wherein said therapeutically effective amount ranges from about 0.1ug/kg to about 1 mg/kg.

15. Use according to any of claims 10 to 14, wherein said metabolic disorder is the prediabetic state of impaired glucose tolerance.

16. Use of a polypeptide selected from the group consisting of the polypeptides of claim 1 for the manufacture of a medicament for stimulating insulin release in a glucose-dependent manner in a mammal.

17. Use of a peptide selected from the group consisting of SEQ ID NOs: 47, 72-82 and 85 for the manufacture of a medicament for treating respiratory disease in a mammal.

## Patentansprüche

1. Polypeptid, ausgewählt aus der Gruppe bestehend aus SEQ ID NOs: 47, 72-82 und 85.

2. Polynucleotid, kodierend für eine Polypeptidsequenz nach Anspruch 1, oder eine degenerierte Variante davon.

3. Vektor, umfassend ein Polynucleotid nach Anspruch 2.

4. Isolierte Wirtszelle, umfassend einen Vektor nach Anspruch 3.

5. Verfahren zum Herstellen eines Polypeptids nach Anspruch 1, umfassend:
a) Kultivieren der Wirtszelle nach Anspruch 4 unter für die Expression des Polypeptids geeigneten Bedingungen; und
b) Gewinnen des Polypeptids aus der Wirtszellkultur.

6. Pharmazeutische Zusammensetzung, umfassend ein Polypeptid nach Anspruch 1 in Kombination mit einem pharmazeutisch annehmbaren Träger.

7. Gentherapiezusammensetzung, umfassend ein Polynucleotid nach Anspruch 2 in Kombination mit einem therapeutisch wirksamen Gentherapievektor.

8. Polypeptid nach Anspruch 1, wobei das Polypeptid durch SEQ ID NO: 72 wiedergegeben wird.

9. Gereinigter Antikörper, welcher spezifisch an das Polypeptid nach Anspruch 1 bindet.

10. Verwendung eines PACAP R3 Agonisten, ausgewählt aus der Gruppe bestehend aus SEQ ID NOs: 47, 72-82 und 85, für die Herstellung eines Medikaments zum Behandeln einer Stoffwechselstörung in einem Säuger.

11. Verwendung nach Anspruch 10, wobei der PACAP R3 Agonist eine wenigstens ungefähr 100-fache Selektivität für PACAP R3 gegenüber PACAP R2 oder PACAP R1 aufweist.

12. Verwendung nach einem der Ansprüche 10 bis 11, wobei der R3-Agonist das Polypeptid von SEQ ID NO: 72 ist.

13. Verwendung nach einem der Ansprüche 10 bis 12, wobei die Stoffwechselstörung Typ 2 Diabetes ist.

14. Verwendung nach einem der Ansprüche 10 bis 13, wobei die therapeutisch wirksame Menge im Bereich von ungefähr 0,1 µg/kg bis ungefähr 1 mg/kg liegt.

15. Verwendung nach einem der Ansprüche 10 bis 14, wobei die Stoffwechselstörung der prädiabetische Zustand einer gestörten Glucosetoleranz ist.

16. Verwendung eines Polypeptids, ausgewählt aus der Gruppe bestehend aus den Polypeptiden nach Anspruch 1, für die Herstellung eines Medikaments zum Stimulieren der Insulinfreisetzung auf eine glucoseabhängige Weise in einem Säuger.

17. Verwendung eines Peptids, ausgewählt aus der Gruppe bestehend aus SEQ ID NOs: 47, 72-82 und 85, für die Herstellung eines Medikaments zum Behandeln einer Atemwegserkrankung in einem Säuger.

## Revendications

1. Polypeptide choisi dans le groupe consistant en SEQ ID NOs: 47, 72-82 et 85.

2. Polynucléotide codant une séquence polypeptidique selon la revendication 1, ou son variant dégénéré.

3. Vecteur comprenant un polynucléotide selon la revendication 2.

4. Cellule hôte isolée comprenant un vecteur selon la revendication 3.

5. Procédé de production d'un polypeptide selon la revendication 1 comprenant :
a) la culture de la cellule hôte selon la revendication 4 dans des conditions appropriées pour l'expression dudit polypeptide ; et
b) la récupération du polypeptide à partir de la culture de la cellule hôte.

6. Composition pharmaceutique comprenant un polypeptide selon la revendication 1 en combinaison avec un véhicule pharmaceutiquement acceptable.

7. Composition de thérapie génique comprenant un polynucléotide selon la revendication 2 en combinaison avec un vecteur de thérapie génique thérapeutiquement efficace.

8. Polypeptide selon la revendication 1, dans lequel ledit polypeptide est représenté par SEQ ID NO: 72.

9. Anticorps purifié qui se lie spécifiquement au polypeptide selon la revendication 1.

10. Utilisation d'un agoniste de PACAP R3 choisi dans le groupe constitué de SEQ ID NOs: 47, 72-82 et 85 pour la fabrication d'un médicament pour le traitement d'un trouble du métabolisme chez un mammifère.

11. Utilisation selon la revendication 10, dans laquelle l'agoniste de PACAP R3 a au moins une sélectivité de 100 fois pour PACAP R3 par rapport à PACAP R2 ou PACAP R1.

12. Utilisation selon l'une quelconque des revendications 10 à 11, dans laquelle l'agoniste de R3 est le polypeptide de SEQ ID NO: 72.

13. Utilisation selon l'une quelconque des revendications 10 à 12, dans laquelle ledit trouble du métabolisme est le diabète de type 2.

14. Utilisation selon l'une quelconque des revendications 10 à 13, dans laquelle ladite quantité thérapeutiquement efficace varie d'environ 0,1 µg/kg à environ 1 mg/kg.

15. Utilisation selon l'une quelconque des revendications 10 à 14, dans laquelle ledit trouble du métabolisme est l'état prédiabétique d'intolérance au glucose.

16. Utilisation d'un polypeptide choisi dans le groupe constitué des polypeptides selon la revendication 1 pour la fabrication d'un médicament pour stimuler la libération d'insuline de manière dépendante du glucose chez un mammifère.

17. Utilisation d'un peptide choisi dans le groupe constitué de SEQ ID NOs: 47, 72-82 et 85 pour la fabrication d'un médicament pour le traitement d'une maladie respiratoire chez un mammifère.
